# EUROPEAN PATENT APPLICATION

(11) **EP 2 423 181 A1**
(43) Date of publication of application: **29.02.2012**
(21) Application number: 10382208.6
(22) Date of filing: 28.07.2010
(51) Int. Cl.: C07C 217/48

(54) **Multitarget substituted biphenyl diol derivatives**

(71) Applicant: PROUS INSTITUTE FOR BIOMEDICAL RESEARCH S.A., 08028 Barcelona (ES)
(72) Inventor: Munoz, Rosa, E- 08028, Barcelona (ES); Garcia-Delgado, Noemí, E- 08028, Barcelona (ES); Flores, Ramón, E- 08028, Barcelona (ES); Serradell, Neus, E- 08028, Barcelona (ES); Prous, Josep R., E- 08028, Barcelona (ES)
(74) Representative: ABG Patentes, S.L.

(57) **Abstract**

New multitarget biphenyl diol compounds and their use as a medicament to suppress tumor growth and/or prevent recurrence or metastasis or to treat a cancer selected from the group consisting of brain, bone, colon, ovary, pancreas, prostate, skin, lung, and head and neck cancer, melanoma and hematological malignancies or to treat nononcological indications such as skin inflammation, atopic dermatitis, or actinic keratosis or to treat autoimmune disorders such as multiple sclerosis, psoriatic arthritis, rheumatoid arthritis, scleroderma, systemic lupus, erythematosus, dermatomyositis, Sjögren's syndrome and Behçet's disease or to treat pain, selected from the group consisting of chronic, cancer, postoperative and neuropathic pain or to simultaneously treat cancer and cancer-related pain and inflammation.

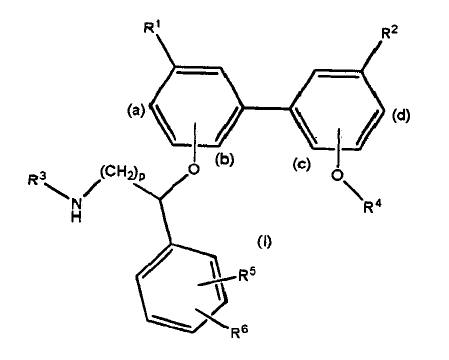

## Description

### FIELD OF THE INVENTION

The present invention relates to multitarget biphenyl diol compounds and their use in the preparation of pharmaceutical compositions for treating cancer, pain, rheumatoid arthritis, and inflammatory skin conditions.

### BACKGROUND OF THE INVENTION

According to the World Health Organization (WHO), cancer is a leading cause of death worldwide. The disease accounted for 7.4 million deaths (or around 13% of all deaths worldwide) in 2004. The main types of cancer leading to overall cancer mortality each year are: lung (1.3 million deaths/year), stomach (803,000 deaths), colorectal (639,000 deaths), liver (610,000 deaths), and breast cancer (519,000 deaths). Deaths from cancer worldwide are projected to continue to rise, with an estimated 12 million deaths in 2030.

Cancer is primarily treated with one or a combination of three types of therapies: surgery, radiation, and chemotherapy. The adverse effects of systemic chemotherapy are feared the most by patients; these can include nausea, vomiting, and a wide range of complications that impact on the patient's quality of life.

Pain is a prevalent symptom in cancer patients, affecting up to 50% of patients undergoing active cancer treatment and up to 90% of those with advanced disease (Cherny, N.I. et al. Schmerz, 1994, 8, 195-209; Cleeland, C.S. et al. N Engl J Med, 1994, 330, 592-596). In addition to the physical and emotional suffering due to the diagnosis of cancer, patients are subjected to additional discomfort and symptoms associated with both the disease and its treatment. While an increasing amount of clinical and basic scientific research has focused on these issues, our understanding of the mechanisms underlying cancer-related symptoms remains incomplete. The International Association for the Study of Pain defines pain as "an unpleasant sensory and emotional experience associated with actual or potential tissue damage, or described in terms of such damage". Cancer pain can be managed effectively through relatively simple means in up to 90% of the 8 million Americans who have cancer or a history of cancer. Unfortunately, pain associated with cancer is frequently undertreated.

Depression is a disabling illness that affects around 15-25% of cancer patients (Patrick, D.L. et al. J Natl Cancer Inst Monogr, 2004, (32), 9-16). Men and women with cancer are affected equally. People who face a diagnosis of cancer will experience different levels of stress and emotional upset. Depression increases cancer patients' risk of dying.

Stressful events have been linked to depression (Hammen, C. Annu Rev Clin Psychol, 2005, 1, 293-319; Mazure, C.M. Clin Psychol, 2006, 5, 291-313; Monroe, S.M., Simons, A.D. Psychol Bull, 1991, 110, 406-425). Approximately 20-25% of people who experience major stressful events develop depression (van Praag, H.M. et al. Stress, the Brain and Depression, Cambridge: Cambridge University Press, 2004). Experimental work with animals has provided support for a contribution of stress to the initiation, growth, and metastasis of selected tumors (Cohen, S. et al. JAMA, 2007, 298, 1685-1687). Prospective studies linking stress and cancer incidence have been reported (Duijts, S.F.A. et al. Int J Cancer, 2003, 107, 1023-1029; Heffner, K.L. et al. Brain Behav Immun, 2003, 17, S109-S111; Turner-Cobb, J.M. et al. In: Ader, R., Felten, D.L., Cohen, N. [Eds.], Psychoneuroimmunology, 3rd Ed. New York: Academic Press, 2001, 565-582). Different disorders emerge in individuals who have been exposed to stress (Lupien, S.J. et al. Nat Rev Neurosci, 2009, 10, 434-445). Stress resilience involves adaptive changes in specific neural circuits, neuromodulation levels, and molecular pathways (Feder, A. et al. Nat Rev Neurosci, 2009, 10, 446-457).

A further problem of anticancer drugs in general, and of antiproliferative drugs in particular, is the tendency of tumor cells to develop resistance towards such anticancer drugs. As a possible solution to this problem, it has been suggested (Guarino, M.G. J Cell Physiol, 2010, 223(1), 14-26) to use a combination therapy comprising tyrosine-protein kinase Src and EGFR (erbB-1) inhibitors.

The search for multitarget drugs has always been an attractive strategy for modulating efficacy and selectivity and to overcome the drawbacks of toxicity and resistance. Molecules such as honokiol and magnolol, with multiple pharmacological properties, display structural features suitable for interaction with various biological targets, thereby producing specific pharmacological effects. Magnolol and honokiol, however, show low inhibitory activity against Src and EGFR kinase.

Honokiol and magnolol have been reported to possess multiple pharmacological actions, including antiplatelet, antibacterial, antitumor, antiangiogenic, anxiolytic, and antidepressant properties (Qiang, L. et al. Arch Pharm Res, 2009, 32(9), 1281-1292; Matsunaga, T. et al. Biochem Biophys Res Commun, 2009, 389(1), 128-132; Gu, W. et al. Xibao Yu Fenzi Mianyixue Zazhi, 2008, 24(6), 620-622; Luo, Y. Bioorg Med Chem Lett, 2009, 19(16), 4702-4705 ;Battle, T.E. et al. Blood, 2005, 106(2), 690-697; Park, E.J. et al. Cancer Lett, 2009, 277(2), 133-140; Sun, L. et al. Yiyao Daobao, 2008, 27(1), 69-71; Deng, J. et al. Liver Int, 2008, 28(10), 1458-1464; Yang, G. et al. Sichuan Daxue Xuebao, Yixueban, 2008, 39(4), 558-562).

Honokiol and magnolol, natural products isolated from *Magnolia* spp., have been described in multiple publications (see, for example: Patocka, J. et al. J Appl Biomed, 2006, 4(4), 171-178; Fried, L.E., Arbiser, J.L. Antioxid Redox Signal, 2009, 11 (5), 1139-1148) to have antitumor activity..

Certain analogues or derivatives of honokiol and/or magnolol have been reported to have cell proliferation-inhibitory activity (Luo, Y. et al. Bioorg Med Chem Lett, 2009, 19, 4702-4705; Kong, Z.-L. et al. Bioorg Med Chem Lett, 2005, 15, 163-166), effects on the CNS (Lee, Y.K. et al. Neurochem Res, 2009, 34, 2251-2260), anti-inflammatory activity (Oh, J.H. et al. Chem Biol Interact, 2009, 180, 506-514; Zhou, H.Y. et al. Eur J Pharmacol, 2008, 586, 340-349), inhibitory activity against cyclooxygenase (Schuehly, W. et al. Bioorg Med Chem, 2009, 17, 4459-4465; Schuehly, W. et al. Inflammopharmacology 2009, 17, 106-110), and inhibitory activity against leukotriene LTB₄ and LTC₄ formation (Schuehly, W. et al. Bioorg Med Chem, 2009, 17, 4459-4465; Hamasaki, Y. et al. Planta Med, 1999, 65, 222-226).

Analogues of both compounds have been reported in patents and current literature. The following patent references disclose biphenyl derivatives for treating cancer: WO 95/29682, WO 2006/107451, WO 2008/099994, and WO 2008/137420. The following patent references disclose biphenyl derivatives with other activities: US 4537996 (antiallergic agents), JP 03027312 A (blood platelet aggregation inhibitors), EP 0382213 B1 (nerve cell-repairing or - protecting activity), JP 070022655 A (agents for repairing or protecting denaturation of peripheral nerves), WO 99/00346 (antianxiety activity), WO 00/40532 (antianxiety activity), JP 2001026537 A and WO 01/03689 (antianxiety activity), WO 2004/072016 and also EP 1593666 (β₃-adrenoceptor agonists), WO 2006/101818 A1 (antibacterial activity), JP 2006347940 A (β-amyloid formation inhibitors), WO 2007/114095 (melanogenesis inhibitors), WO 2006/059714 and US 2007/0232703 (preventing and/or treating bone diseases), WO 2008/102086 (modulating testosterone levels), WO 2009/158467 A2 (phosphodiesterase PDE10 inhibitors), JP 2009114101 (treatment of periodontal diseases), and FR 2933300 (treatment of acne) and CN 1270168, TW 503235, JP 2004292392 (as antioxidants).

Thus, there is a need to discover new anticancer drugs which show a reduced tendency to induce tumor cell resistance, as well as possessing antistress, analgesic and/or anti-inflammatory effects, and/or antidepressant effects.

The compounds of formula (I) have utility in the therapeutic treatment of cancer as a result of their activity as inhibitors of Src and EGFR kinase. Additionally, the compounds show pleiotropic effects which can be explained mechanistically by their actions on different targets, i.e., binding to receptors and inhibition of enzymes. These effects may allow the use of the compounds in the treatment of a variety of other diseases, including pain, rheumatoid arthritis, and inflammatory skin conditions.

### DESCRIPTION OF THE INVENTION

The inventors have obtained new substituted biphenyl diol derivatives showing a dual inhibitory effect against Src and EGFR kinase. This dual activity makes them promising candidates for the treatment of cancer likely to show a reduced tendency to develop cancer cell resistance. Advantageously, these new drugs also possess pain- (COX-1, Formalin test, Hot plate test) and/or inflammation-relieving (5-LO), and/or antidepressant effects.

In one aspect, the present invention provides biphenyl diol compounds of formula (I): 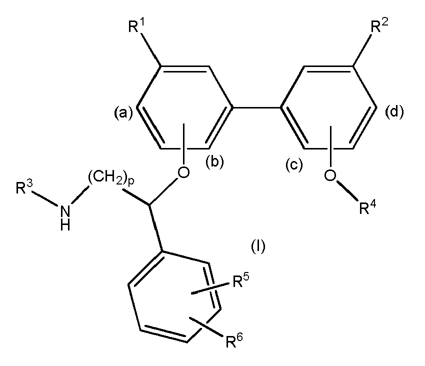 wherein R¹ and R² are independently selected from the group consisting of C₁-C₈ alkyl, C₂-C₈ alkenyl, and C₂-C₈ alkynyl;
R³ is a C₁-C₄ alkyl group;
p is an integer selected from 1, 2, 3, and 4;
both R⁵ and R⁶ are independently selected from hydrogen atom, C₁-C₈ alkyl, - OH, halogen atoms, and -CF₃; or R⁵ and R⁶ together with the phenyl ring to which they are attached form a benzodioxole group;
R⁴ is a group selected from:
a) hydrogen atom, C₁-C₈ alkyl, C₂-C₈ alkenyl, and C₂-C₈ alkynyl, all optionally substituted by one or more groups selected from halogen, -N(R^{a})(R^{b}), - O(C=O)-C₁-C₈ alkylene-N(R^{a})(R^{b}), -O(C=O)-C₁-C₈ alkenylene-N(R^{a})(R^{b}), C₃-C₂₀ cycloalkyl, C₄-C₁₂ cycloalkenyl, -NH-C(=NH)-NH₂, -C(=NH)-NH₂, - (CH₂)ₙ-SO₂-R, -N(R^{a})-S(=O)₂-R, -S(=O)₂-N(R^{a})(R^{b}), -N(R^{a})-C(=O)O-R, - S(=O)-R, -S(=O)₂-R, -N(C₁-C₈ alkyl)-C(=O)N(R^{a})(R^{b}), -NHOH, C₆-C₁₀ aryl, heterocyclyl, and heteroaryl;
b) C₃-C₂₀ cycloalkyl, C₄-C₁₂ cycloalkenyl, and C₆-C₁₀ aryl, all optionally substituted by one or more groups selected from halogen, -OH, -CF₃, -C₁-C₈ alkylene-N(R^{a})(R^{b}), -O(C=O)-C₁-C₈ alkylene-N(R^{a})(R^{b}), C₃-C₂₀ cycloalkyl, C₄-C₁₂ cycloalkenyl, -NH-C(=NH)-NH₂, -C(=NH)-NH₂, -(CH₂)ₙ-S(=O)₂-R, - N(R^{a})S(=O)₂-R, -S(=O)₂-N(R^{a})(R^{b}), -N(R^{a})-C(=O)O-R, -S(=O)-R, -S(=O)₂-R, -N(C₁-C₈ alkyl)-C(=O)N(R^{a})(R^{b}), -NHOH, C₆-C₁₀ aryl, heterocyclyl, and heteroaryl;
c) heterocyclyl and heteroaryl, both optionally substituted by one or more groups selected from -OH, -CF₃, C₁-C₈ alkyl, C₂-C₈ alkenyl, C₂-C₈ alkynyl, C₃-C₂₀ cycloalkyl, C₄-C₁₂ cycloalkenyl, halogen, -C₁-C₈ alkylene-N(R^{a})(R^{b}), -(CH₂)ₙ-S(=O)₂-R, C₆-C₁₀ aryl-C₁-C₆ alkyl, and phenyl optionally substituted by one or more groups selected from C₁-C₈ alkyl, perfluoro-C₁-C₈ alkyl, C₁-C₈ alkoxy, hydroxy-C₁-C₈ alkyl, halogen, -OH, -S-R, or benzyloxy;
d) -C(=O)N(R^{a})(R^{b});
e) -C(=O)OR^{c};
f) -C(=O)R^{c};
g) -P(=O)(OR^{d})(OR^{e});
h) -P(=O)(OR^{d})-CH₂-P(=O)(OR^{d})(OR^{e});
i) a sugar moiety selected from: 1-O-β-D-glucopyranosyl, 1-O-β-D-galactopyranosyl, and 1-O-α-D-glucopyranosyl-α-D-glucopyranosyl;
j) an aminoacid moiety derived from a naturally occurring aminoacid;
n is an integer selected from 1 to 6;
wherein R is a hydrogen atom or a group selected from C₁-C₈ alkyl, C₂-C₈ alkenyl, C₂-C₈ alkynyl, C₃-C₂₀ cycloalkyl, C₆-C₁₀ aryl, and C₆-C₁₀ aryl-C₁-C₈ alkyl;
R^{a} and R^{b} are independently selected from hydrogen atom, C₁-C₈ alkyl, C₂-C₈ alkenyl, C₂-C₈ alkynyl, C₃-C₂₀ cycloalkyl, C₄-C₁₂ cycloalkenyl, C₆-C₁₀ aryl, heterocyclyl, heteroaryl, and C₂-C₈ acyl, all of them optionally substituted by one or more groups selected from halogen, C₁-C₈ alkoxy, -S-C₁-C₈ alkyl, C₆-C₁₀ aryl, heteroaryl, -NH₂, -NH-C₁-C₈ alkyl, -N(C₁-C₈ alkyl)₂, -S(=O)₂-C₁-C₈ alkyl, -S(=O)-C₁-C₈ alkyl, -C(=O)OH, -C(=O)O-C₁-C₈ alkyl and -C(=O)-C₁-C₈ alkyl;
or R^{a} and R^{b} together with the N to which they are attached form a saturated, monounsaturated or polyunsaturated 5,6-membered nitrogenated heterocycle, which may optionally contain another heteroatom selected from O, N, and S; R^{c}, R^{d}, and R^{e} are independently selected from hydrogen atom, C₁-C₈ alkyl, C₂-C₈ alkenyl, C₂-C₈ alkynyl, C₂-C₈ acyl, C₃-C₂₀ cycloalkyl, C₄-C₁₂ cycloalkenyl, C₆-C₁₀ aryl, heterocyclyl and heteroaryl, all of them optionally substituted by one or more groups selected from halogen, -OH, C₁-C₈ alkoxy, -S-C₁-C₈ alkyl, C₆-C₁₀ aryl, heteroaryl, -NH₂, -NH-C₁-C₈ alkyl, -N(C₁-C₈ alkyl)₂, -S(=O)₂-C₁-C₈ alkyl, - S(=O)-C₁-C₈ alkyl, -C(=O)OH, -C(=O)O-C₁-C₈ alkyl and -C(=O)-C₁-C₈ alkyl;
wherein either the substituent -O-CH(substituted phenyl)-(CH₂)p-NH-R³ occupies the carbon atom marked as (a) while the substituent -OR⁴ occupies the carbon atom marked as (c), or the substituent -O-CH(substituted phenyl)-(CH₂)ₚ-NH-R³ occupies the carbon atom marked as (b) while the substituent - OR⁴ occupies either the carbon atom marked as (c) or the carbon atom marked as (d);
or a pharmaceutically acceptable salt or stereoisomer thereof.

In one embodiment, the present invention provides a compound of formula (I) in salt form wherein the secondary amino group of the group -O-CH(substituted phenyl)-(CH₂)ₚ-NH-R³ is further protonated (-NH₂⁺-) and the compound further comprises a pharmaceutically acceptable counter-anion. The term "pharmaceutically acceptable counter-anion" is meant to designate an anion derived from a pharmaceutical acceptable mono- or polyprotic acid which maintains the electric neutrality of the compound of formula (I).The compounds, according to this particular embodiment, have improved solubility and have a lower distribution coefficient or log D_{octanol/water} which often translates into a superior bioavailability.

In another embodiment, the present invention provides a compound of formula (I) wherein R⁴ is selected from:
a) hydrogen atom, C₁-C₈ alkyl, C₂-C₈ alkenyl and C₂-C₈ alkynyl, all of which are optionally substituted by one or more groups selected from halogen, - N(R^{a})(R^{b}), -O(C=O)-C₁-C₈ alkylene-N(R^{a})(R^{b}), C₃-C₂₀ cycloalkyl, -NHOH, C₆-C₁₀ aryl, heterocyclyl and heteroaryl;
b) C₃-C₂₀ cycloalkyl, C₄-C₁₂ cycloalkenyl and C₆-C₁₀ aryl, all optionally substituted by one or more groups selected from halogen, -C₁-C₈-alkylene-N(R^{a})(R^{b}), -O(C=O)-C₁-C₈ alkylene-N(R^{a})(R^{b}), C₃-C₂₀ cycloalkyl, -NHOH, C₆-C₁₀ aryl, heterocyclyl and heteroaryl;
c) heterocyclyl and heteroaryl, both optionally substituted by one or more groups selected from C₁-C₈ alkyl, C₃-C₂₀ cycloalkyl, halogen, -C₁-C₈ alkylene-N(R^{a})(R^{b}) and phenyl optionally substituted by one or more groups selected from C₁-C₈ alkyl, -CF₃, C₁-C₈ alkoxy, hydroxy-C₁-C₈ alkyl, halogen and -OH;
d) -C(=O)N(R^{a})(R^{b});
e) -C(=O)OR^{c};
f) -C(=O)R^{c};
g) -P(=O)(OR^{d})-CH₂-P(=O)(OR^{d})(OR^{e});
h) a sugar moiety selected from: 1-O-β-D-glucopyranosyl, 1-O-β-D-galactopyranosyl, and 1-O-α-D-glucopyranosyl-α-D-glucopyranosyl;
R^{a} and R^{b} are independently selected from hydrogen atom, C₁-C₈ alkyl, C₃-C₂₀ cycloalkyl, C₆-C₁₀ aryl, and C₂-C₈ acyl all of them optionally substituted by one or more groups selected from halogen, C₁-C₈ alkoxy, -S-C₁-C₈ alkyl, C₆-C₁₀ aryl and heteroaryl;
or R^{a} and R^{b} together with the N to which they are attached form a saturated, monounsaturated or polyunsaturated 5,6-membered nitrogenated heterocycle, which may optionally contain another heteroatom selected from 0, N, and S; wherein R^{c}, R^{d}, and R^{e} are independently selected from hydrogen atom, C₁-C₈ alkyl, C₂-C₈ alkenyl, C₃-C₂₀ cycloalkyl, C₆-C₁₀ aryl, heterocyclyl, and heteroaryl all of them optionally substituted by one or more groups selected from halogen, -OH, C₁-C₈ alkoxy, -S-C₁-C₈ alkyl, -C(=O)O-C₁-C₈ alkyl, C₆-C₁₀ aryl and heteroaryl;

In another embodiment, the present invention provides a compound of formula (I) wherein p has a value selected from 1, 2 and 3, preferably 2 and R³ is a group selected from methyl, ethyl, *n*-propyl and isopropyl, preferably a methyl group.

In another embodiment, the present invention provides a compound of formula (I) wherein R¹ and R² are independently selected from the group consisting of allyl, 2-propenyl, *n*-propyl, methyl, isopropyl, *tert*-butyl, and ethynyl, preferably allyl and *n*-propyl, and most preferably allyl.

In another embodiment, the present invention provides a compound of formula (I) wherein R⁴ is selected from:
a) hydrogen atom, C₁-C₈ alkyl and C₂-C₈ alkenyl, both of them optionally substituted by one or more groups selected from halogen, -N(R^{a})(R^{b}), - O(C=O)-C₁-C₈ alkylene-N(R^{a})(R^{b}), C₃-C₂₀ cycloalkyl, -NHOH, C₆-C₁₀ aryl, heterocyclyl and heteroaryl;
b) C₃-C₂₀cycloalkyl and C₆-C₁₀ aryl, both optionally substituted by one or more groups selected from halogen, -C₁-C₈-alkylene-N(R^{a})(R^{b}), -O(C=O)-C₁-C₈ alkylene-N(R^{a})(R^{b}), C₃-C₂₀ cycloalkyl, -NHOH, C₆-C₁₀ aryl, heterocyclyl and heteroaryl;
c) heterocyclyl and heteroaryl, both optionally substituted by C₁-C₈ alkyl, C₃-C₂₀ cycloalkyl, halogen and phenyl optionally substituted by one or more groups selected from C₁-C₈ alkyl, halogen and -OH;
d) -C(=O)N(R^{a})(R^{b});
e) -C(=O)R^{c};
f) -P(=O)(OR^{d})-CH₂-P(=O)(OR^{d})(OR^{e});
g) a sugar moiety selected from: 1-O-β-D-glucopyranosyl and 1-O-α-D-glucopyranosyl-α-D-glucopyranosyl;
wherein R^{a}, R^{b}, R^{c}, R^{d} and R^{e} are as defined above
or a pharmaceutically acceptable salt or stereoisomer thereof.

In another embodiment, the present invention provides a compound of formula (I) wherein R⁴ is selected from:
a) hydrogen atom, C₁-C₈ alkyl and C₂-C₈ alkenyl, both optionally substituted by one or more groups selected from halogen, -N(R^{a})(R^{b}), -O(C=O)-C₁-C₈ alkylene-N(R^{a})(R^{b}), C₃-C₂₀ cycloalkyl, -NHOH and C₆-C₁₀ aryl;
b) C₃-C₂₀cycloalkyl and C₆-C₁₀ aryl, both optionally substituted by one or more groups selected from halogen, -C₁-C₈-alkylene-N(R^{a})(R^{b}), -O(C=O)-C₁-C₈ alkylene-N(R^{a})(R^{b}), C₃-C₂₀ cycloalkyl, -NHOH and C₆-C₁₀ aryl;
c) -C(=O)N(R^{a})(R^{b});
d) -C(=O)R^{c};
e) -P(=O)(OR^{d})-CH₂-P(=O)(OR^{d})(OR^{e});
f) a sugar moiety selected from: 1-O-β-D-glucopyranosyl and 1-0-α-D-glucopyranosyl-α-D-glucopyranosyl;
wherein R^{a}, R^{b}, R^{c}, R^{d}, and R^{e} are as defined above;
or a pharmaceutically acceptable salt or stereoisomer thereof.

In another embodiment, the present invention provides a compound of formula (I) wherein R⁴ is selected from:
a) hydrogen atom;
b) C₁-C₄ alkyl optionally substituted by one or more halogen atoms;
c) -C(=O)R^{c};
wherein R^{c} is a C₁-C₈ alkyl group.

In yet another embodiment, the present invention provides a compound of formula (I) wherein R⁴ is selected from hydrogen atom, methyl and ethyl.

In yet another embodiment, the present invention provides a compound of formula (I) wherein R⁴ is selected from:
a) hydrogen atom, C₁-C₈ alkyl and C₂-C₈ alkenyl, both optionally substituted by one or more groups selected from -N(R^{a})(R^{b}), C₆-C₁₀ aryl, heterocyclyl and heteroaryl;
b) C₃-C₂₀cycloalkyl and C₆-C₁₀ aryl, both optionally substituted by one or more groups selected from C₆-C₁₀ aryl, heterocyclyl and heteroaryl;
c) heterocyclyl and heteroaryl, both optionally substituted by C₁-C₈ alkyl, C₃-C₂₀ cycloalkyl, halogen and phenyl optionally substituted by one or more groups selected from C₁-C₈ alkyl, halogen and -OH;
wherein R^{a} and R^{b} are as defined above;
or a pharmaceutically acceptable salt or stereoisomer thereof.

In yet another embodiment, the present invention provides a compound of formula (I) selected from:
- 5,5'-Diallyl-2'-trifluoromethoxy-2-(3-methylamino-1-phenylpropoxy)biphenyl.
- 5,5'-Diallyl-2-(4-methoxy-4-oxobut-2-enoyloxy)-2'-(3-methylamino-1-phenylpropoxy)biphenyl.
- 5,5'-Dimethyl-2-methoxy-2'-(3-methylamino-1-phenylpropoxy)biphenyl.
- 2'-[3-(Methylamino)-1-phenylpropoxy]-5,5'-di(prop-1-enyl)biphenyl-2-ol.
- 2'-(3-Methylamino-1-phenylpropoxy)-3,5'-di(prop-1-enyl)biphenyl-4-ol.
- 2-Methoxy-4'-(3-methylamino-1-phenylpropoxy)-3',5-di(prop-1-enyl)biphenyl.
- 5,5'-Diallyl-2'-[3-(methylamino)-1-phenylpropoxy]biphenyl-2-ol.
- 5,5'-Diallyl-2-methoxy-2'-[3-(methylamino)-1-phenylpropoxy]biphenyl.
- 5,5'-Diallyl-2-ethoxy-2'-[3-(methylamino)-1-phenylpropoxy]biphenyl.
- 3,5'-Diallyl-2'-[3-(methylamino)-1-phenylpropoxy]biphenyl-4-ol.
- 3',5-Diallyl-4'-[3-(methylamino)-1-phenylpropoxy]biphenyl-2-ol.
- 4'-[3-(Methylamino)-1-phenylpropoxy]-3',5-dipropylbiphenyl-2-ol.
- 3',5-Diallyl-4'-ethoxy-2-[3-(methylamino)-1-phenylpropoxy]biphenyl mixture with 3,5'-diallyl-2'-ethoxy-4-[3-(methylamino)-1-phenylpropoxy]biphenyl.
- 5,5'-Diallyl-2'-[3-(methylamino)-1-phenylpropoxy]biphenyl-2-yl 2-propylpentanoate.

In another aspect the present invention provides pharmaceutical compositions comprising a compound as defined above and one or more pharmaceutically acceptable carriers.

In another aspect, the present invention provides a compound as defined above for use as a medicament, in particular for use in suppressing tumor growth and/or preventing recurrence or metastasis or in the treatment of a cancer selected from the group consisting of brain, bone, colon, ovary, pancreas, prostate, skin, lung, and head and neck cancer, melanoma and hematological malignancies, or in the treatment of nononcological indications such as skin inflammation, atopic dermatitis, or actinic keratosis or in the treatment of autoimmune disorders such as multiple sclerosis, psoriatic arthritis, rheumatoid arthritis, scleroderma, systemic lupus, erythematosus, dermatomyositis, Sjögren's syndrome and Behçet's disease or in the treatment of pain, selected from the group consisting of chronic, cancer, postoperative and neuropathic pain or for the simultaneous treatment of cancer and cancer-related pain and inflammation.

In yet another aspect the present invention comprises a combination comprising a compound as defined above and another anticancer agent, in particular wherein the additional anticancer agent is selected from the group consisting of paclitaxel, cisplatin, carboplatin, trastuzumab, lapatinib, tamoxifen, letrozole, gefitinib, anastrozole, everolimus, irinotecan, or temozolomide.

In yet another aspect, the present invention comprises a combination comprising a compound as defined above and another active ingredient selected from the group consisting of *O*⁶-benzylguanine, glucosamine, valproic acid, butyric acid, metformin, or dichloroacetate.

Compounds of formula (I) are also much more potent and have a broader spectrum of activity against tumor cells than magnolol and honokiol. In particular, they show dual inhibition of both Src and EGFR kinase.

In addition, the compounds of formula (I) are active against several other biological targets, including the enzymes cyclooxygenase-1 (COX-1), 5-lipoxygenase (5-LO), serine/threonine-protein kinases, in particular Akt, and peptide receptors, in particular gonadotrophin-releasing hormone receptor (GnRH, LHRH), as well as in cellular processes such as autophagy, apoptosis induction, and angiogenesis inhibition.

In particular, the compounds of the present invention show dual inhibitory activity against Src and EGFR kinases. At least some of the compounds of the present invention also show agonist activity at the GnRH receptor, in addition to inhibitory activity against COX-1. It is believed that these pharmacological effects, in particular their ability to inhibit both Src and EGFR kinases is the basis for their activity against tumor cells.

Prostate and breast cancer cells usually metastasize to bone, resulting in the development of bone lesions that cause pain and patient morbidity.

Experimental studies have shown that Src, a non-receptor tyrosine-protein kinase, is an important signaling molecule during the processes of osteoclast-mediated bone resorption, tumor growth, and metastasis. As such, Src represents a promising target for the treatment of advanced metastatic prostate and breast cancer (Saad, F., Lipton, A. Cancer Treat Rev, 2010, 36, 177-184; Zhang, X.H.F., Wang, Q. et al., Cancer Cell, 2009, 16(1), 67-78).

Targeted treatment with EGFR inhibitors produces resistance, possibly mediated by increased Src activity, which in turn leads to enhancement of EGFR activation. Treatment with compounds within the scope of the invention prevents resistance to EGFR inhibitors (Guarino, M. J Cell Physiol, 2010, 223, 14-26).

*In vitro* cancer screening of the compounds of formula (I) has shown that they are active against different human tumor cell lines, including leukemia, melanoma, and cancer of the lung, colon, brain, ovary, breast, prostate, and kidney. *In vivo* efficacy of the compounds of formula (I) has been proven using tumor xenograft models.

Chemical structure-activity relationship studies of compounds of formula (I) have allowed the enhancement of activity of certain compounds against selected targets. Thus, certain compounds of formula (I) were found to be highly active against inflammatory pain or in other nononcological conditions.

Quite unexpectedly, compounds within the scope of the invention are very active against inflammatory pain due to tumorigenesis.

Compounds within the scope of the invention show highly improved pharmacokinetic and pharmacological profiles when compared to magnolol and honokiol, thus presenting good potential for therapeutic intervention.

Lipophilicity has been correlated to various drug properties, affecting permeability, absorption, distribution, plasma protein binding, metabolism, elimination, and toxicity. In contrast to honokiol and magnolol, compounds claimed in the invention, and more particularly those wherein the nitrogen atom in the group -O-CH(phenyl)-(CH₂)ₚ-NH-R³ is protonated and the compound further comprises a pharmaceutically acceptable counter-anion present an optimal log D_{octanol/water} (range 0-3), indicating good aqueous solubility, permeability, oral absorption, and blood-brain barrier penetration. Experimental studies in murine models have confirmed the results. The log D_{octanol/water} of magnolol and honokiol is 5.25, which denotes a poor solubility and pharmacokinetic profile.

| **Compound** | **logD_{octanol/water}** |
|---|---|
| Magnolol | 5.25 |
| Honokiol | 5.25 |
| Example1 | 1.31 |
| Example 5 | 0.85 |

Biological tests performed with the compounds of the examples have also shown that they have the following advantages:
1. Good aqueous solubility, permeability, oral absorption, and blood-brain barrier penetration.
2. Broad-spectrum activity against tumor cells, mainly consisting of brain, bone, breast, colorectal, ovary, prostate, skin, lung, and head and neck cancer, melanoma, leukemia, and lymphoma, with prominent activity against invasion, other tumor progression-related events and the development of metastasis.
3. Dual mechanism of antitumor activity (inhibition of both Src and EGFR kinase), thereby avoiding the development of resistance.
4. Autophagy and apoptosis induction.
5. Ability to prevent breast and prostate cells from metastasizing to bone which results in the development of bone lesions that cause pain and patient morbidity.
6. Dual COX/5-LO inhibition providing anti-inflammatory activity.
7. Induction of mild analgesic and antidepressant activity.
8. Lower acute and chronic toxicity than currently used antineoplastic agents.

Therapeutic indications for the compounds of formula (I) include leukemia, melanoma, and cancers of the lung, colon, brain, ovary, breast, prostate, and kidney, as well as pain, musculoskeletal disorders (rheumatoid arthritis, inflammatory pain), and inflammatory skin conditions.

Compounds of formula (I) are administered for human or veterinary use orally, parenterally, subcutaneously, rectally, topically, by inhalation, and locally.

The present invention also provides for compounds of formula (I) pharmaceutical compositions employing such compounds and methods of using such compounds alone or in combination with other therapeutic agents for treating, preventing, or delaying the progression of the above-mentioned diseases.

In addition, in accordance with the present invention, the preparation of a pharmaceutical composition is provided for treating cancer and related inflammation and pain as defined above and hereafter, wherein a therapeutically effective amount of a combination of a compound of formula (I) and another anticancer agent and/or another type of therapeutic agent is given to enhance the antitumor effects or avoid possible side effects. Antitumor agents include paclitaxel, cisplatin, carboplatin, trastuzumab, lapatinib, tamoxifen, letrozole, gefitinib, anastrozole, everolimus, irinotecan, or temozolomide. Also, combinations of two or more compounds of formula (I) are included. The pharmaceutical composition is administered to a human patient in need of treatment.

The compounds of formula (I) of the invention can be prepared as shown in the following reaction schemes and the description thereof wherein temperatures are expressed in degrees Celsius.

Compounds of formula (Ib) can be prepared as shown in Scheme 1 by treatment of compounds of formula (Ia) with compounds of formula (III) or by treatment of compounds of formula (II) with compounds of formula (V).

Compounds of formula (Ia) can be prepared by treatment of compounds of formula (IV) with compounds of formula (V).

Compounds of formula (II) can be prepared by treatment of compounds of formula (IV) with compounds of formula (III).

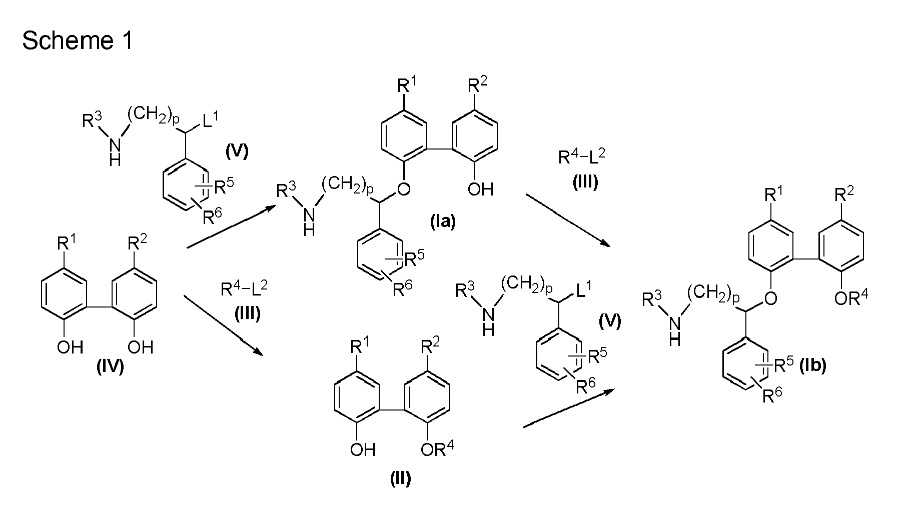

In the above depicted scheme when L¹ and L² represent a halogen atom or when R⁴ is a -C(=O)R^{c} group and L² a halogen or a -OC(=O)R^{c} group, the reactions are performed using a base such as K₂CO₃, Cs₂CO₃, NEt₃, pyridine, DMAP, DIPEA, 1*H*-tetrazole, DBU, NaH, NaOH, or *t*-BuOK, in a solvent such as DMF, CH₂Cl₂, THF, ACN, toluene, xylene, hexane, acetone, EtOH, or H₂O at a temperature between 0 and 100 °C.

When L¹ and L² represent an-OH group, the reactions are performed using triphenylphosphine and an alkyl azadicarboxylate such as DIAD or DEAD employing a solvent such as toluene or THF. Alternatively, compounds of formula (III) or (V) can react with alkyl-S(=O)₂X or aryl-S(=O)₂X (where X = halide) such as methanesulfonyl chloride or 4-toluenesulfonyl chloride in the presence of a base such as NEt₃, followed by the reaction of these intermediates with the desired reactants using a base such as NEt₃ or pyridine and in a solvent such as CH₂Cl₂, THF, or DMF.

When R⁴ is a sugar moiety the compounds can be conveniently obtained following this scheme using a compound of formula (III) wherein L² represents an -OC(=O)CH₃ or -OC(=NH)CCl₃ group. In this case the corresponding reaction is performed using a Lewis acid such as BF₃·Et₂O, SnCl₄, or TMSOTf employing a solvent such as CH₂Cl₂, DCE, THF, ACN or toluene.

Compounds of formula (Ib) and compounds of formula (II), where R⁴ is a -CH₃ group, can be obtained by reaction of compounds of formula (Ia) and compounds of formula (IV), respectively with an ethereal solution of diazomethane at 20 °C for 24 h.

Compounds of formula (Ib) and compounds of formula (II), where R⁴ is a -CH₃, - CH₂CH₃ or -CH₂CH₂CH₃ group can be obtained by reaction of compounds of formula (Ia) and compounds of formula (IV), respectively with a sulfate of formula R⁴-O-S(=O)₂-O-R⁴ in the presence of a base such as K₂CO₃, NaOH, or NaH in a solvent such as DMF, THF, ACN, acetone, or H₂O at 20 °C.

When R¹ and R² are not identical, the above-mentioned reactions yield a mixture of compounds which may be separated after each step of the reaction, or alternatively at the end of the synthetic path, by conventional separation techniques such as chromatographic, enzymatic, or crystallographic methods.

Compounds of formula (Ie) can be prepared as shown in Scheme 2 by treatment of compounds of formula (VI) with compounds of formula (V) or treatment of compounds of formula (Ic) with compounds of formula (III).

Compounds of formula (If) can be prepared as shown in Scheme 2 by treatment of compounds of formula (VII) with compounds of formula (V) or treatment of compounds of formula (Id) with compounds of formula (III).

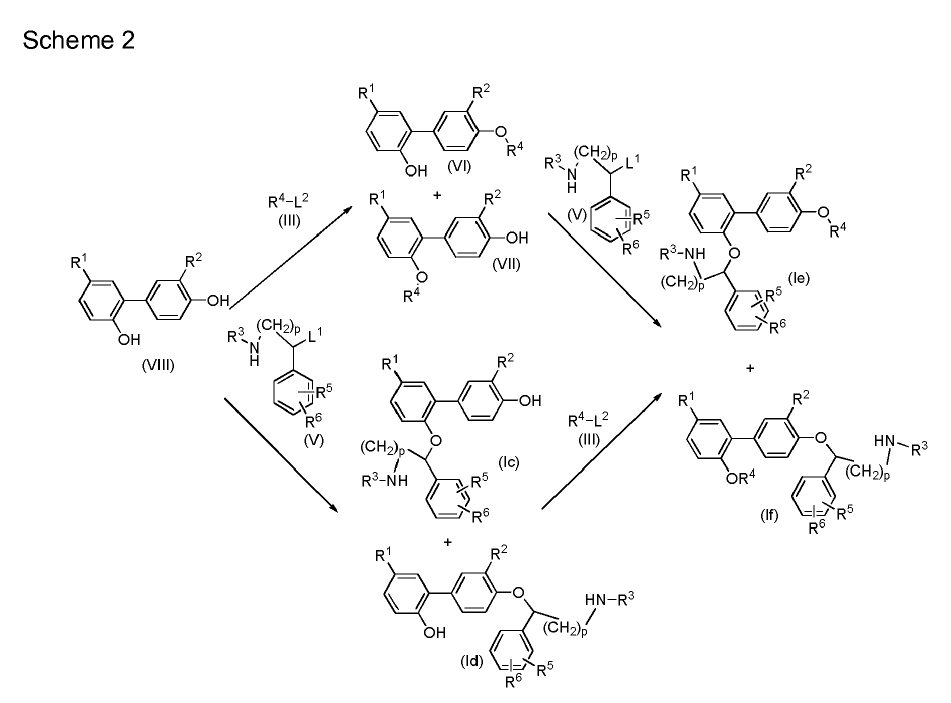

A mixture of compounds of formula (VI) and (VII) can be prepared by treatment of compounds of formula (VIII) with compounds of formula (111). The individual compounds can be isolated from the mixture by chromatographic, enzymatic, or crystallographic methods.

Similarly, a mixture of compounds of formula (Ic) and (Id) can be prepared by treatment of compounds of formula (VIII) with compounds of formula (V). The individual compounds can be isolated from the mixture by chromatographic, enzymatic, or crystallographic methods.

In the above-depicted scheme, when L¹ and L² represent a halogen atom or when R⁴ is a -C(=O)R^{c} group and L² a halogen or an -OC(=O)R^{c} group, the reactions are performed using a base such as K₂CO₃, CS₂CO₃, NEt₃, pyridine, DMAP, DIPEA, 1*H-*tetrazole, DBU, NaH, NaOH, or t-BuOK, in a solvent such as DMF, CH₂Cl₂, THF, ACN, toluene, xylene, hexane, acetone, EtOH, or H₂O at a temperature between 0 and 100 °C.

When L¹ and L² represent an -OH group, the reactions are performed using triphenylphosphine and an alkyl azadicarboxylate such as DIAD or DEAD employing a solvent such as toluene or THF. Alternatively, compounds of formula (III) or (V) can react with alkyl-S(=O)₂X or aryl-S(=O)₂X (where X = halide), such as methanesulfonyl chloride or 4-toluenesulfonyl chloride, in the presence of a base such as NEt₃, followed by the reaction of these intermediates with the desired reactants using a base such as NEt₃ or pyridine in a solvent such as CH₂Cl₂, THF, or DMF.

When R⁴ is a sugar moiety the compounds can be conveniently obtained following this scheme using a compound of formula (III) wherein L² represents an -OC(=O)CH₃ or -OC(=NH)CCl₃ group. In this case the reactions are performed using a Lewis acid such as BF₃·Et₂O, SnCl₄ or TMSOTf employing a solvent such as CH₂Cl₂, DCE, THF, ACN or toluene.

Mixtures of compounds of formulae (VI) and (VII) and mixtures of compounds of formulae (Ie) and (If), where R⁴ is a group -CH₃, can be obtained by reaction of compounds of formula (VIII) and compounds of formula (Ic) and (Id), respectively, with an ethereal solution of diazomethane at 20 °C for 24 h. The individual compounds can be isolated from the mixture by chromatographic, enzymatic, or crystallographic methods.

Mixtures of compounds of formulae (VI) and (VII) and mixtures of compounds of formulae (Ie) and (If), where R⁴ is a -CH₃, -CH₂CH₃ or -CH₂CH₂CH₃ group can be obtained by reaction of compounds of formula (VIII) and compounds of formula (Ic) and (Id), respectively, with a sulfate of formula R⁴-O-S(=O)₂-O-R⁴ in the presence of a base such as K₂CO₃, NaOH, or NaH in a solvent such as DMF, THF, ACN, acetone, or H₂O at 20 °C.

Biphenyl derivatives represented by the general formula (I) also include biphenyl derivatives represented by the general formula (Ig).

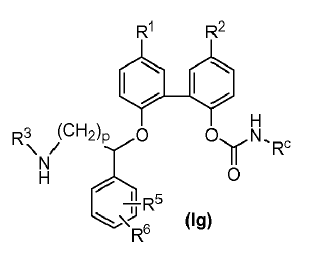

Compounds of formula (Ig) can be prepared as shown in Scheme 3 by treatment of compounds of formula (Ia) with compounds of formula (IX) or compounds of formula (X) with compounds of formula (V).

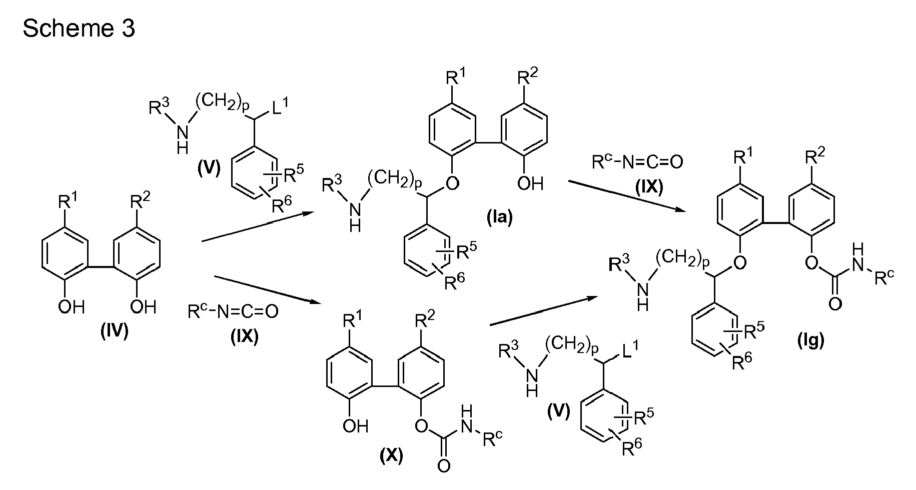

In one synthetic route compounds of formula (Ia), previously described, are reacted with isocyanates of formula (IX) in the presence of a base such as NEt₃, pyridine, K₂CO₃, or NaH in a solvent such as CH₂Cl₂, THF, diethyl ether, toluene, or ACN at 20 °C to give compounds of formula (Ig).

In another synthetic route compounds of formula (X) can be prepared by treatment of compounds of formula (IV) with isocyanates of formula (IX) in the presence of a base such as NEt₃, pyridine, K₂CO₃, or NaH in a solvent such as CH₂Cl₂, THF, diethyl ether, toluene, or ACN at 20 °C.

In the above depicted scheme, when L¹ represents a halogen atom, the reactions are performed using a base such as K₂CO₃, NEt₃, pyridine, or NaH in a solvent such as DMF, CH₂Cl₂, THF, ACN, or acetone at 20 °C to provide compounds of formula (Ia) or (Ig).

When L¹ represents an -OH group, the reactions are performed using triphenylphosphine and an alkyl azodicarboxylate such as DIAD or DEAD employing a solvent such as toluene or THF. Alternatively, compounds of formula (V) can react with alkyl-S(=O)₂X or aryl-S(=O)₂X (where X = halide) such as methanesulfonyl chloride or 4-toluenesulfonyl chloride in the presence of a base such as NEt₃, followed by the reaction of these intermediates with compounds of formula (X) to provide compounds of formula (Ig) or with compounds of formula (IV) to provide compounds of formula (Ia) using a base such as NEt₃ or pyridine in a solvent such as CH₂Cl₂, THF, or DMF.

Compounds of formula (III), (IV), (V), (VIII), and (X) are either commercially available or readily prepared by standard methods known to those skilled in the art (Sartori, G. et al. Tetrahedron Lett, 1992, 33, 2207-2210; Sartori, G. et al. J Org Chem, 1993, 58, 7271-7273; Kong, Z.-L. et al. Bioorg Med Chem Lett, 2005, 15, 163-166; Ishii, Y. et al. Tetrahedron Lett, 2006, 47, 8221-8225).

The following abbreviations are employed herein:
- ACN = acetonitrile
- DBU = 1,8-diazabicyclo[5.4.0]undec-7-ene
- DCE = dichloroethane
- DEAD = diethyl azodicarboxylate
- DIAD = diisopropyl azodicarboxylate
- DIPEA = diisopropylethylamine
- DMAP = 4-dimethylaminopyridine
- DMSO = dimethylsulfoxide
- DMF = dimethylformamide
- EtOAc = ethyl acetate
- EtOH = ethanol
- Et₂O = diethyl ether
- g = gram(s)
- MeOH = methanol
- mg = milligram(s)
- mL = milliliter
- mmol = millimoles
- mol = moles
- mp = melting point
- NEt₃ = triethylamine
- THF = tetrahydrofuran
- TMSOTf = trimethylsilyl trifluoromethanesulfonate

The term "alkyl" as employed herein alone or as part of another group designates both straight- and branched-chain saturated hydrocarbons containing 1 to 20 carbons, preferably 1 to 10 carbons, and more preferably 1 to 8 carbons. Examples of alkyl groups are methyl, ethyl, *n*-propyl, isopropyl, *n-*butyl, isobutyl, *tert*-butyl, pentyl, hexyl, isohexyl, heptyl, 4,4-dimethylpentyl, octyl, 2,2,4-trimethylpentyl, nonyl, decyl, undecyl, dodecyl, and the various branched-chain isomers thereof.

The term "alkenyl" as employed herein alone or as part of another group includes both straight- and branched-chain hydrocarbons containing 2 to 20 carbons, preferably 2 to 12 carbons, and more preferably 2 to 8 carbons, and includes 1 to 6 double bonds. Examples of alkenyl groups are vinyl, 2-propenyl, 2-butenyl, 3-butenyl, 3-pentenyl, 4-pentenyl, 2-hexenyl, 3-hexenyl, 2-heptenyl, 4-heptenyl, 3-octenyl, 3-nonenyl, 4-decenyl, 3-undecenyl, and 4,8,12-tetradecatrienyl.

The term "alkynyl" as employed herein alone or as part of another group includes both straight- and branched-chain hydrocarbons containing from 2 to 20 carbons, preferably 2 to 12 carbons, and more preferably 2 to 8 carbons, and includes 1 to 6 triple bonds and optionally 1 to 3 double bonds. Examples of alkynyl groups are 2-propynyl, 3-butynyl, 4-pentynyl, 2-hexynyl, 4-heptynyl, 3-octynyl, 3-nonynyl, 4-decynyl, 3-undecynyl, and 4-dodecynyl.

The term "alkanoyl" as used herein alone or as part of another group refers to an alkyl group attached to a carbonyl group. Thus, a Cₙ alkanoyl groups is a Cₙ₋₁ alkyl group attached to a carbonyl group. Examples of alkanoyl groups are acetyl, propionyl, and butyroyl.

The term "cycloalkyl" as employed herein alone or as part of another group includes saturated cyclic hydrocarbon groups containing 1 to 3 rings, including monocyclic alkyl, bicyclic alkyl, and tricyclic alkyl systems and containing a total of 3 to 20 carbon atoms, preferably 3 to 10 carbons, forming part of the ring system. Examples of cycloalkyl groups are cyclopropyl, cyclobutyl, cyclopentyl, cyclohexyl, cycloheptyl, cyclooctyl, cyclodecyl, cyclododecyl, adamantly, and bicyclo[3.3.3]undecanyl. The term "cycloalkyl" additionally embraces the above-mentioned groups optionally carrying one or more substituents selected from C₁-C₈ alkyl, C₂-C₈ alkenyl, C₂-C₈ alkynyl, C₃-C₂₀ cycloalkyl, C₄-C₁₂ cycloalkenyl, halogen, -NH-C₁-C₈ alkyl, -(CH₂)ₙ-S(=O)₂-R, C₆-C₁₀ aryl-C₁-C₈ alkyl, C₂-C₈ acyl, or a phenyl group optionally substituted by C₁-C₈ alkyl, -CF₃, C₁-C₈ alkoxy, hydroxy-C₁-C₈ alkyl, halogen, -OH, C₁-C₈ alkylthio, and benzyloxy wherein R is a hydrogen atom or a group selected from C₁-C₈ alkyl, C₂-C₈ alkenyl, C₂-C₈ alkynyl, C₃-C₂₀cycloalkyl, C₆-C₁₀ aryl, and C₆-C₁₀ aryl-C₁-C₈ alkyl.

The term "cycloalkenyl" as employed herein alone or as part of another group includes partially unsaturated cyclic hydrocarbon groups containing 1 or 2 double bonds and having 1 to 3 rings, including monocyclic alkyl, bicyclic alkyl, and tricyclic alkyl systems, containing a total of 4 to 12 carbons, preferably 5 to 10 carbons, as part of the ring system. Examples of cycloalkenyl groups include, but are not limited to, cyclobutenyl, cyclopentenyl, cyclohexenyl, cycloheptenyl, cyclooctenyl, cyclohexanediyl, and cycloheptanediyl. The term "cycloalkenyl" additionally embraces the above-mentioned groups optionally carrying one or more substituents selected from C₁-C₈ alkyl, C₂-C₈ alkenyl, C₂-C₈ alkynyl, C₃-C₂₀ cycloalkyl, C₄-C₁₂ cycloalkenyl, halogen, -NH-C₁-C₈ alkyl, - (CH₂)ₙ-S(=O)₂-R, C₆-C₁₀ aryl-C₁-C₈ alkyl, C₂-C₈ acyl, or a phenyl group optionally substituted by C₁-C₈ alkyl, -CF₃, C₁-C₈ alkoxy, hydroxy-C₁-C₈ alkyl, halogen, -OH, C₁-C₈ alkylthio, and benzyloxy wherein R is a hydrogen atom or a group selected from C₁-C₈ alkyl, C₂-C₈ alkenyl, C₂-C₈ alkynyl, C₃-C₂₀ cycloalkyl, C₆-C₁₀ aryl, and C₆-C₁₀ aryl-C₁-C₈ alkyl.

The term "aryl" as employed herein alone or as part of another group refers to monocyclic or bicyclic aromatic groups containing 6 to 10 carbons in the ring portion, such as phenyl or naphthyl (including 1-naphthyl and 2-naphthyl), and may optionally include 1 to 3 additional fused carbocyclic rings, such as cycloalkyl. Examples of aryl groups are phenyl, 1-naphthyl, 2-naphthyl, and tetrahydronaphthyl. The term "aryl" additionally embraces the above-mentioned groups optionally carrying one or more substituents selected from C₁-C₈ alkyl, C₂-C₈ alkenyl, C₂-C₈ alkynyl, C₃-C₂₀ cycloalkyl, C₄-C₁₂ cycloalkenyl, halogen, - NH-C₁-C₈ alkyl, -(CH₂)ₙ-S(=O)₂-R, C₆-C₁₀ aryl-C₁-C₈ alkyl, C₂-C₈ acyl or a phenyl group optionally substituted by C₁-C₈ alkyl, -CF₃, C₁-C₈ alkoxy, hydroxy-C₁-C₈ alkyl, halogen, -OH, C₁-C₈ alkylthio and benzyloxy wherein R is a hydrogen atom or a group selected from C₁-C₈ alkyl, C₂-C₈ alkenyl, C₂-C₈ alkynyl, C₃-C₂₀cycloalkyl, C₆-C₁₀ aryl, and C₆-C₁₀ aryl-C₁-C₈ alkyl.
The terms "arylalkyl", "arylalkenyl", and "arylalkynyl" as used alone or as part of another group refer to alkyl, alkenyl, or alkynyl groups as described above having an aryl substituent. Examples of arylalkyl groups are benzyl and phenethyl.
The term "halogen" as used herein alone or as part of another group refers to chlorine, bromine, fluorine, and iodine.

The term "heterocyclyl" as employed herein alone or as part of another group refers to a 5-, 6-, or 7-membered saturated or partially unsaturated ring which includes 1 to 2 heteroatoms, selected from the group consisting of nitrogen, oxygen, and/or sulfur, and such rings optionally fused to an aryl, cycloalkyl, heteroaryl, or heterocyclyl ring. The heterocyclyl group is linked through a carbon atom or a heteroatom. The term "heterocyclyl" additionally embraces the above mentioned groups optionally carrying one or more substituents selected from C₁-C₈ alkyl, C₂-C₈ alkenyl, C₂-C₈ alkynyl, C₃-C₂₀ cycloalkyl, C₄-C₁₂ cycloalkenyl, halogen, -NH-C₁-C₈ alkyl, -(CH₂)ₙ-S(=O)₂-R, C₆-C₁₀ aryl-C₁-C₈ alkyl, C₂-C₈ acyl, or a phenyl group optionally substituted by C₁-C₈ alkyl, -CF₃, C₁-C₈ alkoxy, hydroxy-C₁-C₈ alkyl, halogen, -OH, C₁-C₈ alkylthio, and benzyloxy wherein R is a hydrogen atom or a group selected from C₁-C₈ alkyl, C₂-C₈ alkenyl, C₂-C₈ alkynyl, C₃-C₂ₒ cycloalkyl, C₆-C₁₀ aryl, and C₆-C₁₀ aryl-C₁-C₈ alkyl.

The term "heteroaryl" as employed herein alone or as part of another group refers to a 5- or 6-membered aromatic ring which includes 1, 2, 3, or 4 heteroatoms, selected from the groups consisting of nitrogen, oxygen, or sulfur, and to such rings optionally fused to an aryl, cycloalkyl, heteroaryl, or heterocyclyl ring. The term "heteroaryl" additionally embraces the above mentioned groups optionally carrying one or more substituents selected from C₁-C₈ alkyl, C₂-C₈ alkenyl, C₂-C₈ alkynyl, C₃-C₂₀ cycloalkyl, C₄-C₁₂ cycloalkenyl, halogen, -NH-C₁-C₈ alkyl, -(CH₂)ₙ-S(=O)₂-R, C₆-C₁₀ aryl-C₁-C₈ alkyl, C₂-C₈ acyl or a phenyl group optionally substituted by C₁-C₈ alkyl, -CF₃, C₁-C₈ alkoxy, hydroxy-C₁-C₈ alkyl, halogen, -OH, C₁-C₈ alkylthio and benzyloxy wherein R is a hydrogen atom or a group selected from C₁-C₈ alkyl, C₂-C₈ alkenyl, C₂-C₈ alkynyl, C₃-C₂₀cycloalkyl, C₆-C₁₀ aryl, and C₆-C₁₀ aryl-C₁-C₈ alkyl.
The term "heterocyclylalkyl" as employed herein alone or as part of another group refers to a heterocyclyl group as defined above linked through a C atom or heteroatom to an alkyl chain as defined above.

The term "heteroarylalkenyl" as employed herein alone or as part of another group refers to a heteroaryl group as defined above linked through a C atom or heteroatom to an alkenyl chain as defined above.

The term "heteroarylalkyl" as employed herein alone or as part of another group refers to a heteroaryl group as defined above linked through a C atom or heteroatom to an alkyl chain.

The term "aminoacid moiety derived from a naturally occurring aminoacid" as employed herein refers to an amino acyl moiety -C(=O)-CHG-NH₂ derived from anyone of the following naturally occurring aminoacids: isoleucine, alanine, leucine, asparagine, lysine, aspartic acid, methionine, cysteine, phenylalanine, glutamic acid, threonine, glutamine, tryptophan, glycine, valine, proline, selenocysteine, serine, tyrosine, arginine, histidine, ornithine, and taurine wherein G represents the side chain of the above-mentioned aminoacids.

The term "sugar moiety" as employed herein refers to 1-O-β-D-galactopyranosyl (galactose), 1-O-β-D-glucopyranosyl (glucose), and 1-O-α-D-glucopyranosyl-α-D-glucopyranosyl (trehalose).

Where the compounds of formula (I) are in acid form they may form a pharmaceutically acceptable salt such as hydrochloride, tartrate, etc.

As used herein, the term "pharmaceutically acceptable salt" embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic, or *p*-toluenesulphonic acid. Pharmaceutical acceptable bases include alkali metal (e. g. sodium or potassium) and alkali earth metal (e. g. calcium or magnesium) hydroxides and organic bases, such as alkyl amines, arylalkyl amines, and heterocyclic amines.

All stereoisomers of the compounds of this invention are contemplated either alone or as mixtures thereof. The process of preparation can utilize racemates, enantiomers, or diastereomers as starting materials. When diastereomeric or enantiomeric products are prepared, they can be separated by conventional methods, for example chromatographic or functional crystallization.

Due to their pharmacological properties, the compounds of formula (I) may be used for suppressing tumor growth and/or preventing recurrence or metastasis or in the treatment of a cancer selected from the group consisting of brain, bone, colon, ovary, pancreas, prostate, skin, lung, and head and neck cancer, melanoma and hematological malignancies or in the treatment of nononcological indications such as emphysema, multiple sclerosis, skin inflammation, atopic dermatitis, or actinic keratosis or in the treatment of autoimmune disorders such as multiple sclerosis, psoriatic arthritis, rheumatoid arthritis, scleroderma, systemic lupus, erythematosus, dermatomyositis, Sjögren's syndrome and Behçet's disease or in the treatment of pain, selected from the group consisting of chronic, cancer, postoperative and neuropathic pain or for the simultaneous treatment of cancer and cancer-related pain and inflammation.

Where desired, the compounds of formula (I) may be used in combination with one or more other types of anticancer agents and/or one or more other types of therapeutic agents which may be administered orally in the same dosage form, in a separate oral dosage form, or by injection.

The other types of anticancer agents which may be optionally employed in combination with compounds of formula (I) include antimitotic drugs and angiogenesis inhibitors such as taxanes, and platinum compounds such as cisplatin and carboplatin and another anticancer agent or agent to overcome or prevent resistance and side effects or provide additive or synergistic effects such as paclitaxel, cisplatin, carboplatin, trastuzumab, lapatinib, tamoxifen, letrozole, gefitinib, anastrozole, everolimus, irinotecan, or temozolomide.

For suppressing tumor growth and/or preventing recurrence or metastasis O⁶-benzylguanine, glucosamine, valproic acid, butyric acid, metformin, or dichloroacetate may also be combined with compounds of formula (I).

Drugs of the present invention may be administered orally, parenterally, subcutaneously, rectally, topically, by inhalation, and locally. Any administration method commonly used for drugs, such as tablets, coated tablets, capsules, solution (including nanoemulsion), syrup, powder, suppository, cream, and ointment, may be used. The pharmaceutical composition can be formulated employing conventional liquid or solid vehicles or diluents and pharmaceutical additives according to the desired mode of administration.

### EXAMPLES

The following examples represent specific embodiments of the present invention.

### Example 1

### 5,5'-Diallyl-2'-[3-(methylamino)-1-phenylpropoxy]biphenyl-2-ol hydrochloride

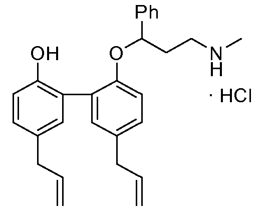

Triphenylphosphine (0.074 g, 0.281 mmol) was dissolved in dry toluene (1.5 mL) and the solution cooled down to 0 °C. A solution of DEAD (∼40% in toluene, 129 µL, 0.281 mmol) was added dropwise and the mixture was stirred for 5 min. α-[2-(Methylamino)ethyl]benzyl alcohol was then added (0.037 g, 0.225 mmol), followed by the addition of magnolol (0.050 g, 0.187 mmol). The solution was stirred at 0 °C for 1 h and at 80 °C for 16 h. The crude mixture was diluted with EtOAc and washed with 10% aqueous HCl (x3). The combined organic fractions were dried (MgSO₄), filtered, and evaporated to dryness. The resulting syrup was purified by column chromatography (SiO₂, EtOAc/MeOH 0-20%). The desired compound was obtained as a yellow solid in 55% yield (0.046 g, 0.102 mmol).
¹H NMR (400 MHz, CD₃OD) δ 7.34 - 7.18 (m, 5H), 7.10 (dd, *J* = 8.2, 2.1, 1H), 7.01 - 6.87 (m, 4H), 6.72 (d, *J* = 8.4, 1H), 6.10 - 5.80 (m, 2H), 5.40 (dd, *J* = 8.4, 3.9, 1H), 5.06 (m, 4H), 3.37 (d, *J* = 6.7, 2H), 3.26 (d, *J* = 6.6, 2H), 3.13 - 2.93 (m, 2H), 2.56 (s, 3H), 2.20 - 2.01 (m, 2H).
¹³C NMR (101 MHz, CD₃OD) δ 154.10, 153.90, 141.81, 139.55, 139.13, 134.47, 132.96, 132.80, 132.77, 130.47, 130.14, 129.84, 129.49, 129.20, 128.16, 127.27, 116.99, 116.04, 115.95, 115.90, 80.05, 48.20, 40.51, 40.40, 35.59, 33.99.

### Example 2

### 5,5'-Diallyl-2-methoxy-2'-[3-(methylamino)-1-phenylpropoxy]biphenyl hydrochloride

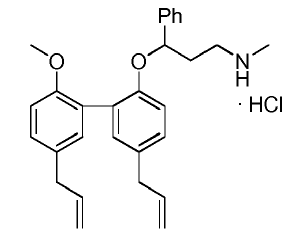

### Method 1

### A. 5,5'-Diallyl-2'-methoxybiphenyl-2-ol

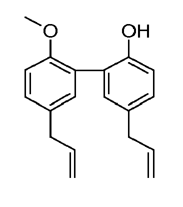

Magnolol (0.100 g, 0.375 mmol) was dissolved in acetone (2 mL) followed by the addition of anhydrous K₂CO₃ (0.078 g, 0.563 mmol) and 18-crown-6 (3 mg, 0.011 mmol). Iodomethane (28 µL, 0.450 mmol) was added dropwise and the solution was stirred for 17 h. The solution was diluted with EtOAc and washed with a saturated aqueous solution of NH₄Cl (x2) and brine (x1). The combined organic fractions were dried (MgSO₄), filtered, and evaporated under vacuum. The desired compound was obtained in 96% yield (0.101 g, 0.360 mmol) as a yellow oil and used without further purification.

### B. 5,5'-Diallyl-2-methoxy-2'-[3-(methylamino)-1-phenylpropoxy]biphenyl hydrochloride

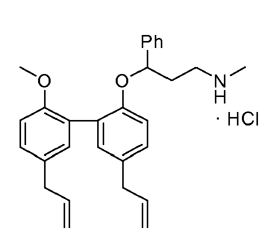

Triphenylphosphine (0.140 g, 0.535 mmol) was dissolved in dry toluene (3 mL) and the solution cooled down to 0 °C. A solution of DEAD (∼40% in toluene, 210 µL, 0.535 mmol) was added dropwise and the mixture was stirred for 5 min. α-[2-(Methylamino)ethyl]benzyl alcohol was then added (0.071 g, 0.428 mmol), followed by the addition of 5,5'-diallyl-2'-methoxybiphenyl-2-ol (0.100 g, 0.356 mmol). The solution was stirred at 0 °C for 1 h and at 80 °C for 16 h. The crude mixture was diluted with EtOAc and extracted with 10% aqueous HCl (x3). The combined organic fractions were dried (MgSO₄), filtered, and evaporated to dryness. The resulting syrup was purified by column chromatography (SiO₂, hexane/EtOAc 0-100% followed by EtOAc/MeOH 0-20%). The desired compound was obtained as a yellow solid in 14% yield (0.023 g, 0.048 mmol).
¹H NMR (400 MHz, CD₃OD) δ 7.38 - 7.16 (m, 6H), 7.04 (dd, *J* = 15.1, 5.2, 2H), 6.91 (d, *J* = 9.7, 2H), 6.66 (d, *J* = 8.1, 1H), 6.10 - 5.80 (m, 2H), 5.30 (dd, *J* = 7.8, 4.6, 1H), 5.18 - 4.92 (m, 4H), 3.79 (s, 3H), 3.40 (d, *J* = 6.7, 2H), 3.27 (d, *J* = 6.6, 2H), 2.89 (dd, *J* = 11.9, 7.0, 2H), 2.48 (s, 3H), 2.17 - 2.01 (m, 2H).
¹³C NMR (101 MHz, CD₃OD) δ 157.16, 154.30, 141.98, 139.39, 139.21, 134.15, 134.13, 133.78, 132.73, 132.60, 130.42, 130.11, 129.88, 129.39, 129.21, 127.20, 116.08, 115.89, 115.66, 112.95, 79.46, 56.78, 47.84, 40.49, 40.40, 35.69, 33.89.

### Method 2

### A. (1-Bromo-3-chloropropyl)benzene

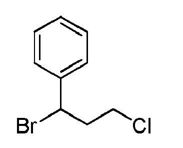

3-Chloro-1-phenyl-1-propanol (1.71 g, 10 mmol) was dissolved in dry Et₂O (27 mL) and purged with N₂. A solution of PBr₃ (0.63 mL, 6.6 mmol) in dry Et₂O (27 mL) was added dropwise very slowly. The reaction was stirred for 2 h at room temperature, followed by washing with 5% aqueous sodium acetate (x2) and brine (x1). The organic fraction was dried (MgSO₄), filtered, and the solvent evaporated to dryness. The resulting crude mixture was purified by column chromatography (SiO₂, hexane/EtOAc) to obtain the desired compound as a yellow oil in 45% yield (1.06 g, 4.54 mmol).

### B. 5,5'-Diallyl-2'-methoxybiphenyl-2-ol

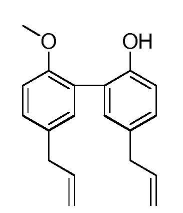

A freshly distilled ethereal solution of diazomethane was added to magnolol (0.820 g, 3.02 mmol). The mixture was stirred for 24 h and the light yellow solution was evaporated to dryness. The resulting crude mixture was purified by column chromatography (SiO₂, hexane/EtOAc) to yield the desired compound as a light yellow oil (95% yield, 0.807 g, 2.87 mmol).

### C. 5,5'-Diallyl-2-(3-chloro-1-phenylpropoxy)-2'-methoxybiphenyl

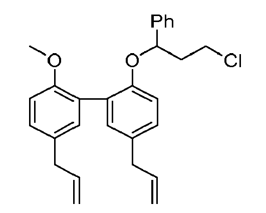

A solution of 5,5'-diallyl-2'-methoxybiphenyl-2-ol (0.200 g, 0.71 mmol) in DMF (0.6 mL) was slowly added to a suspension of NaH (0.034 g, 0.78 mmol, 55% in mineral oil) in dry DMF (0.6 mL). The mixture was stirred for 30 min at room temperature, cooled down to 0 °C, followed by slow addition of a solution of (1-bromo-3-chloropropyl)benzene in dry DMF (0.3 mL). The reaction was stirred at room temperature for 60 h and quenched with EtOH (0.75 mL). The solution was diluted with EtOAc and washed with brine (x2), aqueous 2M NaOH (x2) and brine (x2). The organic fraction was dried (MgSO₄), filtered, and the solvent evaporated to dryness. The resulting crude mixture was purified by column chromatography (SiO₂, hexane/EtOAc) to obtain the desired compound as a yellow oil in 55% yield (0.170 g, 0.39 mmol).

### D.5,5'-Diallyl-2-methoxy-2'-[3-(methylamino)-1-phenylpropoxy]biphenyl hydrochloride

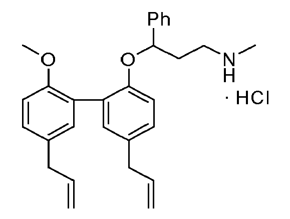

To a solution of 5,5'-diallyl-2-(3-chloro-1-phenylpropoxy)-2'-methoxybiphenyl (0.380 g, 0.8 mmol) in THF (7.2 mL), methylamine (11.9 mL, 138.0 mmol, 40% in water) was added. The mixture was vigorously stirred for 5 days at room temperature and quenched with an aqueous NaOH (2M) solution. The mixture was subsequently extracted with EtOAc (x3) and the combined organic fractions washed with brine (x2). The organic fraction was dried (MgSO4), filtered, and the solvent evaporated to dryness. The resulting crude mixture was purified by column chromatography (SiO2, hexane/EtOAc) to obtain the desired compound as a yellow oil in 34% yield (0.116 g, 0.27 mmol). In order to obtain the hydrochloride salt, the compound was dissolved in dichloromethane and an HCI gas flow was bubbled through the solution for 10 min. The organic solvent was evaporated to dryness under vacuum to afford the compound as a yellow solid.

### Example 3

### 5,5'-Diallyl-2-ethoxy-2'-[3-(methylamino)-1-phenylpropoxylbiphenyl hydrochloride

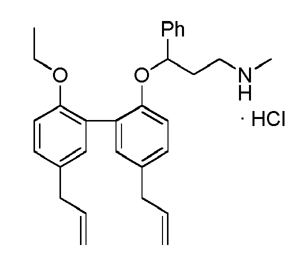

### A. 5,5'-Diallyl-2'-ethoxvbiiphenyl-2-ol

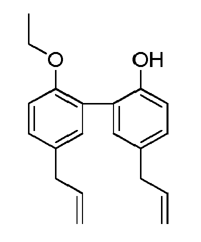

To a solution of magnolol (0.305 g, 1.15 mmol) in acetone (6 mL) anhydrous K₂CO₃ (0.207 g, 1.50 mmol) and 18-crown-6 (9 mg, 0.035 mmol) were added. Iodomethane (86 µL, 1.37 mmol) was added dropwise and the solution was stirred for 48 h. The solution was diluted with EtOAc and washed with saturated NH₄CI (x3) and brine (x3). The combined organic fractions were dried (MgS0₄), filtered, and evaporated under vacuum. The desired compound was obtained in 98% yield (0.334 g, 1.12 mmol) as a yellow oil and used without further purification.

### B. 5,5'-Diallyl-2-ethoxy-2'-[3-(methylamino)-1-phenylpropoxylbiphenyl hydrochloride

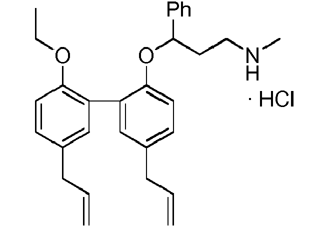

Triphenylphosphine (0.334 g, 1.273 mmol) was dissolved in dry toluene (4 mL) and the solution cooled down to 0 °C. A solution of DEAD (~40% in toluene, 590 µL, 1.273 mmol) was added dropwise and the mixture was stirred for 15 min. a-[2-(Methylamino)ethyl]benzyl alcohol was then added (0.168 g, 1.017 mmol), followed by the dropwise addition of a solution of 5,5'-diallyl-2'-ethoxybiphenyl-2-ol (0.250 g, 0.849 mmol) in dry toluene (1 mL). The solution was stirred at 0 °C for 1 h and at 80 °C for 16 h. The crude mixture was diluted with EtOAc and extracted with 10% aqueous HCI (x3). The combined organic fractions were dried (MgSO₄), filtered, and evaporated to dryness. The resulting syrup was purified by column chromatography (SiO₂, hexane/EtOAc 0-100% followed by EtOAc/MeOH 0-20%). The desired compound was obtained as a yellow solid in 16% yield (0.062 g, 0.140 mmol).
¹H NMR (400 MHz, CD₃OD) δ 7.44 - 7.24 (m, 6H), 7.21 (dd, *J* = 8.3, 2.3 Hz, 1H), 7.11 - 7.02 (m, 2H), 6.99 - 6.89 (m, 2H), 6.67 (d, *J* = 8.4 Hz, 1H), 6.10 - 5.85 (m, 2H), 5.29 (dd, *J* = 7.7, 4.6 Hz, 1H), 5.17 - 4.95 (m, 4H), 4.01 (q, *J* = 7.0 Hz, 2H), 3.41 (d, *J* = 6.7 Hz, 2H), 3.28 (d, *J* = 6.7 Hz, 2H), 2.99 - 2.81 (m, 2H), 2.50 (s, 3H), 2.11 - 1.99 (m, 2H), 1.24 (t, *J* = 7.1, 3H).
¹³C NMR (101 MHz, CD₃OD) δ 156.23, 154.14, 141.80, 139.22, 139.09, 134.00, 133.95, 132.69, 132.57, 130.51, 130.39, 129.95, 129.70, 129.44, 129.26, 129.09, 127.11, 115.96, 115.80, 115.73, 115.05, 79.64, 66.26, 47.74, 40.39, 40.24, 35.45, 33.73, 15.36.

### Example 4

### 3,5'-Diallyl-2'-[3-(methylamino)-1-phenylpropoxy]biphenyl-4-ol hydrochloride

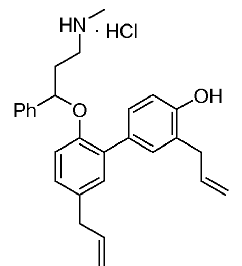

Triphenylphosphine (0.740 g, 2.81 mmol) was dissolved in dry toluene (15 mL) and the solution was cooled down to 0 °C. A solution of DEAD (~40% in toluene, 1.29 mL, 2.81 mmol) was added dropwise and the mixture was stirred for 5 min. a-[2-(Methylamino)ethyl]benzyl alcohol was then added (0.372 g, 2.25 mmol), followed by the addition of honokiol (0.5 g, 1.87 mmol). The solution was stirred at 0 °C for 1 h and at 80 °C for 16 h. The crude mixture was diluted with EtOAc and washed with 10% aqueous HCI (x3). The combined organic fractions were dried (MgS0₄), filtered, and evaporated to dryness. The resulting syrup was purified by column chromatography (SiO₂, EtOAc/MeOH 0-20%). The desired compound was obtained as a yellow solid in 5% yield (0.043 g, 0.095 mmol), and the product described in Example 8 was also isolated.
¹H NMR (200 MHz, CDCl₃) δ 7.42-6.76 (m, 10H), 6.54 (d, *J* = 8.4, 1H), 6.20 - 5.74 (m, 2H), 5.23 - 4.92 (m, 5H), 3.46 (d, *J* = 6.7, 2H), 3.29 (d, *J* = 6.6, 2H), 3.06 - 2.10 (m, 7H).
¹³C NMR (101 MHz, CDCl₃) δ 154.38, 152.43, 139.95, 137.56, 136.97, 133.52, 131.49, 131.21, 130.91, 129.81, 129.12, 128.67, 128.33, 128.05, 127.27, 125.77, 116.14, 115.90, 114.48, 79.83, 47.09, 39.48, 34.59, 34.40, 33.53.

### Example 5

### 3',5-Diallvl-4'-13-(methylamino)-1-phenylpropoxy]biphenyl-2-ol hydrochloride

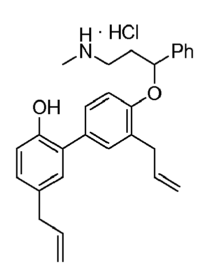

Triphenylphosphine (0.740 g, 2.81 mmol) was dissolved in dry toluene (15 mL) and the solution was cooled down to 0 °C. A solution of DEAD (~40% in toluene, 1.29 mL, 2.81 mmol) was added dropwise and the mixture was stirred for 5 min. a-[2-(Methylamino)ethyl]benzyl alcohol was then added (0.372 g, 2.25 mmol), followed by the addition of honokiol (0.5 g, 1.87 mmol). The solution was stirred at 0 °C for 1 h and at 80 °C for 16 h. The crude mixture was diluted with EtOAc and washed with 10% aqueous HCI (x3). The combined organic fractions were dried (MgS0₄), filtered, and evaporated to dryness. The resulting syrup was purified by column chromatography (SiO₂, EtOAc/MeOH 0-20%). The desired compound was obtained as a yellow solid in 12% yield (0.100 g, 0.265 mmol), and the product described in Example 6 was also isolated.
¹H NMR (400 MHz, CDCl₃) δ 7.34 - 7.16 (m, 7H), 7.07 (dd, *J* = 8.4, 2.1, 1H), 7.00 - 6.88 (m, 2H), 6.63 (d, *J* = 8.6, 1H), 6.07 - 5.84 (m, 2H), 5.33 (s, 1H), 5.13 - 4.94 (m, 4H), 3.45 (d, *J* = 6.3, 2H), 3.29 (d, *J* = 6.7, 2H), 3.01 (t, *J* = 7.3, 2H), 2.56 - 2.24 (m, 5H).
¹³C NMR (101 MHz, CDCl₃) δ 154.63, 151.64, 140.38, 138.27, 137.26, 132.32, 131.45, 130.77, 130.69, 129.41, 129.34, 128.98, 128.58, 128.42, 128.13, 126.21, 116.38, 116.32, 115.91, 113.64, 77.87, 46.83, 39.85, 35.19, 35.13, 33.56, 21.50, 14.65.

### Example 6

### 4'-[3-(Methylamino)-1-phenylpropoxy]-3',5-dipropylbiphenyl-2-ol hydrochloride

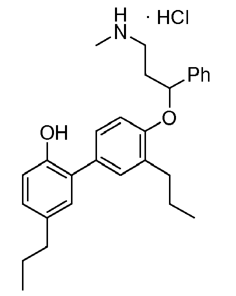

3',5-Diallyl-4'-[3-(methylamino)-1-phenylpropoxy]biphenyl-2-ol hydrochloride (10 mg, 0.022 mmol) was dissolved in 1 mL of MeOH followed by the addition of palladium on charcoal 10% (2 mg). The reaction was stirred for 16 h at room temperature with H₂ (1.013 bar). The reaction was filtered over Celite and washed with MeOH. The solvent was evaporated under vacuum and the desired compound was obtained as a yellow oil in quantitative yield (10 mg, 0.022 mmol).
¹H NMR (400 MHz, CDCl₃) δ 7.29 (m, , 6H), 7.07 - 6.92 (m, 3H), 6.86 (d, *J* = 8.1, 1H), 6.68 (d, *J* = 8.4, 1H), 5.40 (m, 1H), 3.06 (m, 2H), 2.80 - 2.60 (m, 2H), 2.56 (s, 3H), 2.54 - 2.34 (m, 4H), 1.76 - 1.51 (m, 4H), 1.01 (t, *J* = 7.3, 3H), 0.92 (t, *J* = 7.3, 3H).

### Example 7

### 3',5-Diallyl-4'-ethoxy-2-f3-(methylamino)-1-phenylpropoxylbiphenyl hydrochloride mixture with 3,5'-diallyl-2'-ethoxy-4-[3-(methylamino)-1-phenylpropoxy]biphenyl hydrochloride

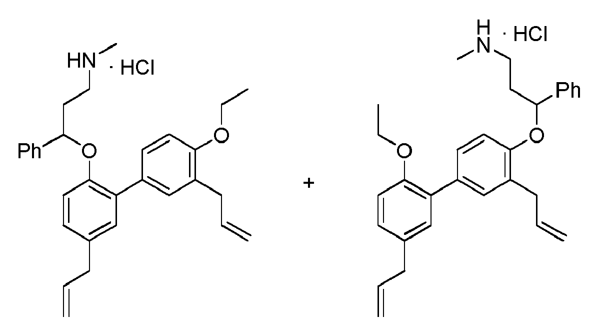

### A. tert-Butyl 3-hydroxy-3-phenylpropyl(methyl)carbamate

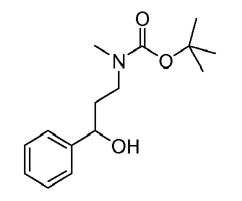

α-[2-(Methylamino)ethyl]benzyl alcohol (0.250 g, 1.51 mmol) was dissolved in THF (2.5 mL) and di-*tert*-butyl dicarbonate (0.332 g, 1.51 mmol) was added to the solution. The reaction was stirred at room temperature until bubbling finished (ca. 1 h). Then the solvent was evaporated and the crude mixture was filtered through a silica plug eluting with EtOAc/MeOH 10%. The compound was obtained as a colorless oil in 87% yield (0.350 g, 1.32 mmol).

### B. tert-Butyl 3-(3',5-diallyl-4'-hydroxybiiphenyl-2-yloxy)-3-phenylpropyl(methyl)carbamate mixture with tert-butyl 3-(3,5'-diallyl-2'-hydroxybiphenyl-4-yloxy)-3-phenylpropyl(methyl)carbamate

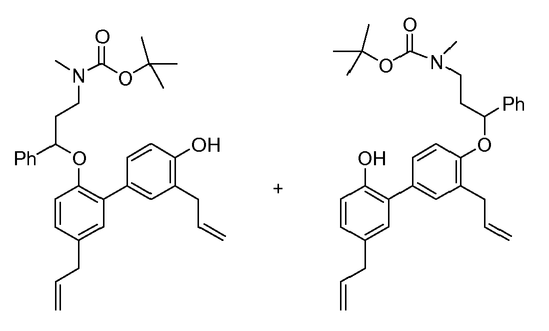

Triphenylphosphine (0.290 g, 1.105 mmol) was dissolved in dry toluene (6 mL) and the solution cooled down to 0 °C. A solution of DEAD (~40% in toluene, 505 µL, 1.105 mmol) was added dropwise and the mixture was stirred for 5 min. *tert-*Butyl 3-hydroxy-3-phenylpropyl(methyl)carbamate was then added (0.235 g, 0.886 mmol), followed by the addition of honokiol (0.196 g, 0.735 mmol). The solution was stirred at 0 °C for 1 h and afterwards 16 h at 80 °C. The crude mixture was extracted with EtOAc/10% aqueous HCl (x3), and the organic fractions were dried (MgS0₄), filtered, and evaporated to dryness. The resulting syrup was purified by column chromatography (Si0₂, EtOAc/MeOH 0-20%). The desired isomeric mixture was obtained as a yellow solid in 71 % yield (0.270 g, 0.525 mmol)

### C. 3',5-Diallyl-4'-ethoxy-2-[3-(methylamino)-1-phenylpropoxy]biphenyl hydrochloride mixture with 3,5'-diallyl-2'-ethoxy-4-[3-(methylamino)-1-phenylpropoxy]biphenyl hydrochloride

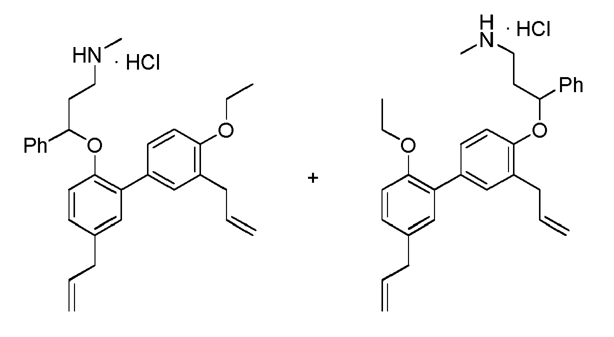

An isomeric mixture of *tert*-butyl 3-(3',5-diallyl-4'-ethoxybiphenyl-2-yloxy)-3-phenylpropyl(methyl)carbamate and *tert*-butyl 3-(3,5'-diallyl-2'-ethoxybiphenyl-4-yloxy)-3-phenylpropyl(methyl)carbamate (0.109 g, 0.201 mmol) was dissolved in methanol (5 mL) and a 37% aqueous HCl solution was also added (1.5 mL). The reaction was stirred for 2 days at room temperature. The organic solvent was evaporated, and the solution was adjusted to pH 8 and extracted with CH₂Cl₂ (x3). The crude mixture was then purified by column chromatography (SiO₂, CH₂Cl₂/MeOH) to afford the desired compound as a yellow solid (mixture of isomers) in 61 % yield (0.059 g, 0.123 mmol).
¹H NMR (400 MHz, CDCl₃) δ 7.44 - 6.98 (m, 8H), 6.97 - 6.78 (m, 2H), 6.65 (dd, *J* = 8.5, 3.8, 1H), 6.17 - 5.83 (m, 2H), 5.30 (dd, *J* = 8.2, 4.3, 0.5H), 5.20 - 4.97 (m, 4.5H), 4.10 (q, *J* = 7.0, 1H), 3.96 (q, *J* = 7.0, 1H), 3.49 (dd, *J* = 22.9, 6.6, 2H), 3.30 (dd, *J* = 12.3, 6.7, 2H), 2.83 (t, *J* = 6.8, 1H), 2.68 - 2.50 (m, 1H), 2.44 (s, 1.5H), 2.22 (s, 1.5H), 2.18 - 1.90 (m, 2H), 1.45 (t, *J* = 7.0, 1.5H), 1.30 (t, *J* = 7.0, 1.5H).
¹³C NMR (101 MHz, CDCl₃) Isomeric mixture 1:1 δ 155.95, 154.51, 154.34, 153.13, 141.79, 141.56, 137.98, 137.81, 137.35, 132.72, 132.37, 131.51, 131.47, 131.30, 131.07, 131.00, 130.91, 130.52, 128.83, 128.78, 128.51, 128.32, 128.20, 128.01, 127.95, 127.89, 127.76, 126.05, 125.97, 115.72, 115.66, 115.63, 115.60, 114.64, 112.92, 112.56, 111.10, 79.51, 77.95, 64.27, 63.88, 48.07, 47.84, 39.61, 39.56, 38.08, 37.31, 35.71, 35.35, 34.89, 34.72, 15.17, 15.01.

### Example 8

### 5,5'-Diallyl-2'-[3-(methylamino)-1-phenylpropoxy]biphenyl-2-yl-2-propylpentanoate hydrochloride

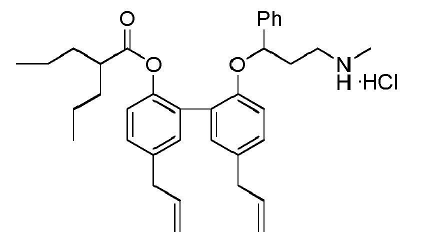

### A. 5,5'-Diallyl-2'-hydroxy-biphenyl-2-yl 2-propylpentanoate

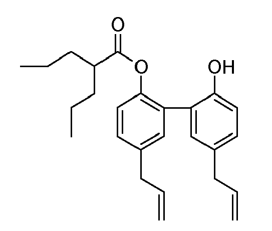

To an ice-cooled solution of magnolol (100 mg, 0.376 mmol) in dichloromethane (2 mL) triethylamine (105 µL, 0.750 mmol), N,N-dimethylaminopyridine (DMAP) (9 mg, 0.08 mmol), and 2,2-di-n-propylacetyl chloride (68 µL, 0.394 mmol) were added. The mixture was allowed to slowly warm to room temperature and was stirred for 20 h. Then, it was diluted with EtOAc and washed with 5% aqueous HCl (x3), 10% aqueous NaHCO₃ (x3) and brine (x3). The organic layer was dried over MgSO₄, filtered, and evaporated to dryness. The resulting syrup (150 mg) was used in the next reaction without further purification.

### B. 5,5'-Diallyl-2'-[3-(methylamino)-1-phenylpropoxy]biphenyl-2-yl-2-propylpentanoate hydrochloride

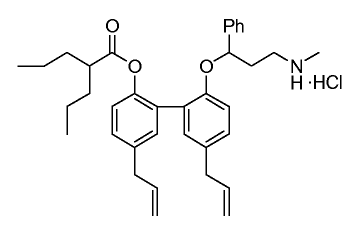

Triphenylphosphine (0.146 g, 0.56 mmol) was dissolved in dry toluene (2 mL) and the solution was cooled down to 0 °C. A solution of DEAD (~40% in toluene, 0.257 mL, 0.56 mmol) was added dropwise and the mixture was stirred for 5 min. α-[2-(Methylamino)ethyl]benzyl alcohol was then added (73 mg, 0.44 mmol), followed by the addition of 5,5'-diallyl-2'-(hydroxybiphenyl-2-yl) 2-propylpentanoate (0.150 g, 0.37 mmol). The solution was stirred at 0 °C for 1 h and at 80 °C for 18 h. The crude mixture was diluted with EtOAc and washed with 10% aqueous HCl (x3). The combined organic fractions were dried (MgSO₄), filtered, and evaporated to dryness. The resulting syrup was purified by column chromatography (SiO₂, hexane/EtOAc 0-40%). The desired compound was obtained as a yellowish solid in 11 % yield (0.24 g, 0.04 mmol).
¹H NMR (400 MHz, CD₃OD) δ 7.51 - 7.17 (m, 8H), 7.07 (d, *J* = 8.2 Hz, 1H), 6.98 - 6.88 (m, 2H), 6.69 (d, *J* = 7.7 Hz, 1H), 6.12 - 6.00 (m, 1H), 5.93 - 5.81 (m, 1H), 5.36 - 5.25 (m, 1H), 5.16 (dd, *J* = 22.0, 13.6 Hz, 2H), 5.09 - 4.94 (m, 2H), 3.50 (d, 6.7 Hz, 2H), 3.27 (d, *J* = 6.7 Hz, 2H), 2.95 - 2.82 (m, 2H), 2.45 - 2.27 (m, 4H), 2.11 - 2.04 (m, 2H), 1.47 - 1.18 (m, 4H), 1.17 - 0.90 (m, 4H), 0.87 - 0.54 (m, 6H).
¹³C NMR (101 MHz, CD₃OD) δ 172.78, 141.83, 139.52, 138.69, 138.56, 134.01, 132.44, 130.11, 129.99, 129.88, 129.23, 127.09, 123.60, 116.69, 116.03, 115.65, 79.96, 46.49, 40.53, 40.24, 35.72, 35.65, 35.45, 33.90, 21.51, 21.40, 14.43, 14.39.

The compounds of examples 9 to 14 are presented in Table 1 (magnolol derivatives) and Tables 2 and 3 (honokiol derivatives). These compounds were prepared employing the procedures of reaction schemes 1 to 3 above. The starting materials for these compounds are either commercially available or readily prepared by standard methods known to those skilled in the art (Sartori, G. et al. Tetrahedron Lett, 1992, 33, 2207-2210; Sartori, G. et al. J Org Chem, 1993, 58, 7271-7273; Kong, Z.-L. et al. Bioorg Med Chem Lett, 2005, 15, 163-166; Ishii, Y. et al. Tetrahedron Lett, 2006, 47, 8221-8225).

**Table 1**

| | | | | | | | |
|---|---|---|---|---|---|---|---|
| 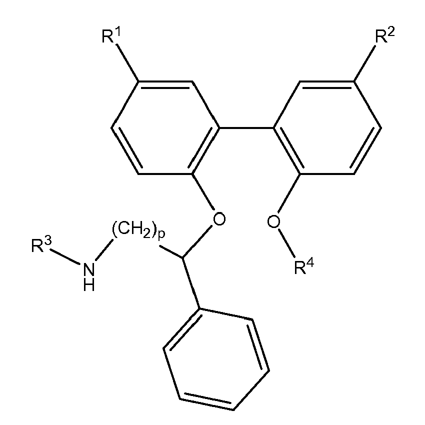 | | | | | | | |

| **Example** | **R¹** | **R²** | **p** | **R³** | **R⁴** | **R⁵** | **R⁶** |
|---|---|---|---|---|---|---|---|
| 9 | 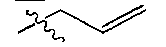 | 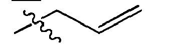 | 2 | Me | CF₃ | H | H |
| 10 | 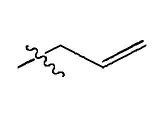 | 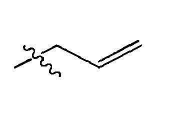 | 2 | Me | 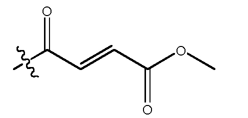 | H | H |
| 11 | Me | Me | 2 | Me | Me | H | H |
| 12 | 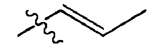 | 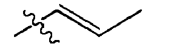 | 2 | Me | H | H | H |

**Table 2**

| | | | | | | | |
|---|---|---|---|---|---|---|---|
| 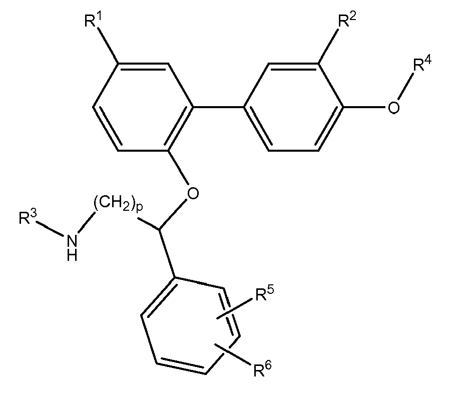 | | | | | | | |

| **Example** | **R¹** | **R²** | **p** | **R³** | **R⁴** | **R⁵** | **R⁶** |
|---|---|---|---|---|---|---|---|
| 13 | 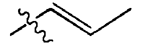 | 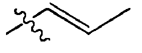 | 2 | Me | H | H | H |

**Table 3**

| | | | | | | | |
|---|---|---|---|---|---|---|---|
| 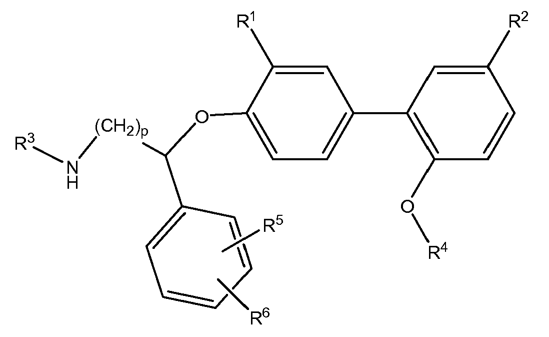 | | | | | | | |

| **Example** | **R¹** | **R²** | **p** | **R³** | **R⁴** | **R⁵** | **R⁶** |
|---|---|---|---|---|---|---|---|
| 14 | 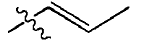 | 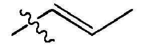 | 2 | Me | Me | H | H |

### BIOLOGICAL ASSAYS

The following examples illustrate the biological activity of compounds of formula (I) and should not be considered as limiting the scope of the invention.

### Inhibition of Protein Kinases

### Src Kinase

Tyosine-protein kinase Src is a cytosol kinase that is activated following the stimulation of different membrane receptors such as the epidermal growth factor receptor (EGFR, erbB-1) and plays a major intracellular role in RNA translation and cell proliferation through the serine/threonine-protein kinase mTOR pathway.
Src kinase regulates cell adhesion, invasiveness and motility in cancer cells, and angiogenesis in the tumor vasculature. Inhibitors of this kinase have been validated for the treatment of cancer, including brain, colorectal, ovary, and prostate cancers, lymphoma, leukemia, melanoma and solid tumors, as well as macular degeneration, and osteoporosis. Src also plays a major role during the processes of osteoclast-mediated bone resorption and metastasis. Thus, Src represents a promising target for the treatment of advanced metastatic cancer of the prostate and breast.

Src protein tyrosine kinase activity was assessed in human recombinant insect Sf9 cells using as a substrate Poly(Glu:Tyr) at 10 µg/mL with preincubation and incubation times of 15 and 5 min, respectively, at 22°C in 50 mM HEPES, pH 7.4, 1 mM DTT, 20 mM MgCl₂ and 0.2 mM Na₃VO₄. An ELISA quantitation method of Poly[Glu:Tyr-P] was employed (Farley, K et al. Anal Biochem, 1992, 203, 151-157).

**Table 4. Inhibition of Src at 10 µM**

| **Compound** | **% Inhibition** |
|---|---|
| Example 1 | 99 |
| Example 2 | 55 |
| Example 5 | > 100 |
| Magnolol | null |
| Honokiol | null |

### EGFR Kinase

EGFR kinase is the prototype of a family of membrane receptor tyrosine-protein kinases which play an important role in the control of cell differentiation, proliferation, and survival.

The use of EGFR inhibitors has been validated for cancer, including biliary, bladder, metastatic breast, cervical, colon, colorectal, esophageal, gastrointestinal, head and neck, kidney, laryngeal, liver, brain, mouth, ovarian, pancreas, prostate, rectal, salivary gland, skin, stomach, thyroid, and adrenocortical cancers, solid tumors, anaplastic astrocytoma, glioblastoma, Hodgkin's lymphoma, and Ménétrier's disease.

EGF receptor tyrosin-protein kinase activity was assessed in human A431 cells using as a substrate Poly(Glu:Tyr) at 10 µg/ml with a preincubation and incubation times of 15 and 60 min, respectively, at 25°C respectively in 50 mM HEPES pH 7.4, 20 mM MgCl₂ and 0.2 mM Na₃VO₄. An ELISA quantification method of Poly[Glu:Tyr-P] was employed (Cheng, K. and Koland, J.G. J Biol Chem, 1996, 271, 311-318).

**Table 5. Inhibition of EGFR at 10 µM**

| **Compound** | **% Inhibition** |
|---|---|
| Example 1 | 72 |
| Example 5 | 99 |
| Magnolol | -26 |
| Honokiol | 7 |

The compound of example 1 inhibits EGFR and Src in vitro by 72% and 99% respectively, and GnRH binding by 50% at 10 µM. Additionally, a slight (but non-significant) inhibition of ERα and ERβ (13 % and 7% respectively, at 10 µM) was observed. These experimental results suggest that the compound of example 1 and related compounds may be useful candidates for the treatment of melanoma and other types of cancer, including prostate, colon, ovary and breast cancers, and other systemic pathologies such as osteoporosis, hypercalcemia and Paget's disease.

### Akt3 (PRKBG) Kinase

Akt3 is a serine/threonine-protein kinase also known as PKB. It is involved in a wide variety of biological processes, including cell proliferation, differentiation, apoptosis and tumorigenesis. Akt3 inhibitors may play a pivotal role in the treatment of malignant tumors.

Akt3 activity was obtained in human recombinant insect cells using Crosstide KK 10 µM as a substrate. The incubation buffer was 20 mM MOPS, pH 7.2, 25 mM β-Glycerophosphate, 1 mM DTT, 5 mM EGTA, 20 mM MgCl₂, 1 mM Na₃VO₄ and the preincubation and incubation times 15 and 30 min, respectively, at 37°C. The quantification of inhibition was assessed by the measure of [³²P]Crosstide KK (Laine, J. Et al. J Biol Chem 2002, 277(5), 3743-3751).

**Table 6. Inhibition of Akt3 (PRKBG) at 10 µM**

| **Compound** | **% Inhibition** |
|---|---|
| Example 1 | 99 |
| Example 5 | 101 |

### CDK2 (CCNA2, cyclin A) Kinase

CDK2 is a protein kinase that is highly active in the G_{1,2}-S phase of the cell cycle. CDK are considered potential targets for anticancer treatment, but selective inhibitors of CDK2 can also induce apoptosis in neutrophil granulocytes which mediates inflammation. Thus, CDK2 inhibitors have a potential role not only in cancer treatment, but also in the treatment of inflammatory diseases such as arthritis.

Human recombinant Sf21 cells were employed to assess the activity on protein serine threonine kinase CDK2/CCNA2 (cdk2/cyclin A). Histone H1 at 100 µg/ml was used as a substrate and the activity was measured by quantification of [³²P]Histone H1 after preincubation and incubation times of 15 and 30 min, respectively, at 37°C in 20 mM MOPS, pH 7.2, 5 mM EGTA, 20 mM MgCl₂, 1 mM DTT, 25 mM β-Glycerophosphate, 1 mM Na₃V0₄ incubation buffer (Gray, N. et al. Curr Med Chem, 1999, 6(9):859-875).

**Table 7. Inhibition of CDK2 at 10 µM**

| **Compound** | **% Inhibition** |
|---|---|
| Example 1 | 92 |
| Example 5 | 98 |

### MAPK1 (ERK2) Kinase

MAPK1 (ERK2, p42MAPK) is a mitogen-activated protein kinase closely related to the metastatic potential of different tumors. Melanoma, breast cancer, medulloblastoma, glioblastoma and acute myelogeneous leukemia, and neurodegenerative diseases such as Alzheimer's or Parkinson's disease, are pathologies where the activation of MAPK1 may play an important physiopathological role.

Protein serine threonine kinase MAPK1(ERK2) was assessed in human recombinant E.Coli using 50 µg/ml myelin basic protein (MBP) as a substrate after preincubation and incubation times of 15 and 30 min, respectively, at 37°C in 20 mM MOPS, pH 7.2, 5 mM EGTA, 20 mM MgCl₂, 1 mM DTT, 25 mM β-glycerophosphate, 1 mM Na₃VO₄ incubation buffer. The inhibitory activity was measured by quantification of [³²P]MBP (Jonhson, G.L. and Lapadat, R. Science 2002, 298(5600), 1911-1912).

**Table 8. Inhibition of MAPK1 at 10 µM**

| **Compound** | **% Inhibition** |
|---|---|
| Example 1 | 58 |
| Example 5 | 80 |

### Antitumor Activity

### Tumor Cell Proliferation Assays

Changes in cell proliferation were detected based on the ability of viable cells to cause alamarBlue® to change its oxidized (non-fluorescent, blue) to a reduced (fluorescent, red) form. The alamarBlue® reaction allows the quantification of cell proliferation and examination of the metabolic activity of viable cells. Test substances were examined for activity on the proliferation of human or murine tumor cell lines at assay concentrations from 0.01 to 100 µM.

Table 9 presents the IC₅₀ values for representative compounds within the scope of this invention.

**Table 9. Inhibition of tumor cell proliferation**

| | **IC₅₀ (µM)** | | | | |
|---|---|---|---|---|---|
| | **Ex. 1** | **Ex. 2** | **Ex. 5** | **Magnolol** | **Honokiol** |
| | | | | | |
| *tumor brain U-87 MG* | 2 | 2.2 | nd | 48 | 56 |
| *tumor colon HCT-116* | 4.3 | 2.1 | nd | 17 | 17 |
| *tumor lung A549* | 5 | 1.3 | 1.2 | 19 | 12 |
| *tumor ovary OVCAR-3* | 4.6 | 2.3 | nd | 23 | 38 |
| *tumor prostate LNCaP-FGC* | 13 | 1.8 | 1.5 | 5.1 | 16 |
| *tumor breast MCF-7* | 1.6 | 2.6 | 4.5 | nd | nd |
| *tumor breast MDA-MB-231* | 3.3 | 2.1 | 2.1 | nd | nd |
| *tumor breast T-47D* | 13 | 12 | 1.6 | nd | nd |
| *tumor liver HepG2* | 1.4 | 3.6 | 1.0 | 12 | 12 |
| *tumor liver Hep 3B* | 5.6 | 3.2 | 1.9 | nd | nd |
| *tumor melanoma B16-FO* | 1.9 | 2.3 | 1.6 | 28 | 30 |
| *tumor leukaemia K562* | 1.9 | 4.2 | 1.4 | 48 | 16 |

| | | | | | |
|---|---|---|---|---|---|
| *nd: not determined* | | | | | |

### Induction of Autophagy and Apoptosis

The induction of autophagy and apoptosis by compounds of formula (I) has been assessed in experimental studies and can be explained by their multitarget mechanism.

### Maximum Tolerated Dose

The Maximum Tolerated Dose (MTD) in mice was determined for selected compounds. The protocol of the studies was in accordance with the standard guidelines for drug development:
(htp://dtp.nci.nih.gov/branches/btb/acute tox.html).

**Table 10. Maximum Tolerated Doses in mice after acute i.p. administration**

| **Compound** | **Maximum Tolerated Dose** |
|---|---|
| Example 1 | 25 mg/kg i.p |
| Example 2 | 50 mg/kg i.p. |
| Example 4 | 30 mg/kg p.o. |
| Example 5 | 25 mg/kg i.p. |

These values are of the same order as the MTD of placlitaxel (20-50 mg/kg i.p. in mice) and higher than the MTD observed with cisplatin (4 mg/kg i.p.). The following comparative chart illustrates the MTD observed with the Examples 1, 2 and 5 and other antineoplasic agents.

### Cyclooxygenase Inhibition

Different forms of cyclooxygenase (COX) are involved in the synthesis of prostaglandins, prostacyclins, and thromboxanes in mammalian cells. The actions of nonsteroidal anti-inflammatory drugs are associated with the inhibition of COX-1 and COX-2.

The COX-1 inhibitory activity of representative compounds of the invention was determined according to previously reported methods. COX-1 activity was assessed in human platelets using 100 µM arachidonic acid as a substrate and preincubation and incubation times of 15 min at 37 °C in HBSS, 15 mM HEPES pH 7.4 . The quantification method used was enzyme immunoassay determination of PGE2 (Chan, C.C. et al. J Pharmacol Exp Ther, 1999, 290, 551-560; Swinney, D.C. et al. J Biol Chem, 1997, 272, 12393-12398; Warner, T.D. et al. Proc Natl Acad Sci U S A, 1999, 96, 7563-7568; Riendeau, D. et al. Can J Physiol Pharmacol, 1997, 75, 1088-1095). COX-2 inhibitory activity was determined in human recombinant insect Sf21 cells according to references (Warner, T.D. et al., Proc Natl Acad Sci, USA. 1999;96(13):7563-7568; Riendeau, D. et al., Can J Physiol Pharmacol, 1997 ;75(9):1088-1095). Briefly, the COX-2 inhibitory activity was assessed on human recombinant insect Sf21 cells using 0.3 µM arachidonic acid as a substrate and preincubation and incubation times of 15 and 5 min, respectively, at 37 °C in 100 mM Tris-HCl, pH 7.7. The quantification method used was enzyme immunoassay determination of PGE2. Results are presented in table 11.

**Table 11. Inhibition of COX-1 and COX-2 at 10 µM**

| **Compound** | **% COX-1 Inhibition** | **% COX-2 inhibition** |
|---|---|---|
| Example 1 | 81 | 0 |
| Example 4 | 81 | 0 |
| Example 5 | 75 | 0 |
| Honokiol | 0 | 79 |
| Magnolol | 21 | 95 |

### Antinociceptive Activity

As COX inhibitors, the compounds are active in in-vivo assays of analgesia, including the acetic acid-, formalin- and phenylquinone- induced writhing tests and the hot plate test.

### Formalin Test

The chemical irritant formalin test is used as a chronic pain model in animals. The s.c. administration of formalin to the paw of a mouse produces a biphasic nociceptive behavioral response, an early response (0-5 min after injection) and a late response (20-30 min after injection) (Hunskaar, S., Fasmer, O.B., Hole, K. Formalin test in mice, a useful technique for evaluating mild analgesics. J Neurosci Methods, 1985 ;14(1):69-76).

*In vivo* studies using the formalin test in mice showed relevant antinociceptive activity for the compound of example 1 (and to a lesser extent for the compound of example 2) at 30 mg/kg i.p in the early and late phases of the experiment, demonstrating similar potency to morphine (10 mg/kg i.p.). In the formalin model, the compound of example 2 showed dose-dependent antinociceptive activity at 10 and 30 mg/kg i.p in the late phase. Table 12 shows the results obtained in the assay. Aspirine and paracetamol are included as external active references in early and late response.

**Table 12. Early and late response inhibition in the formalin test.**

| | **Dose (mg / Kg)** | |
|---|---|---|
| | **Early Response** | **Late Response** |
| | | |
| Example 1 | 10-30 i.p. | 10-30 i.p. |
| | slightly active | active |
| Example 2 | 30 i.p. | 30 i.p. |
| | inactive | active |
| Example 5 | 10-30 i.p. | 10-30 i.p. |
| | slightly active | Active |
| Aspirin | 200-400 p.o. | 300-400 |
| Paracetamol | 200-400 p.o. | 300-400 |
| Morphine | 10 i.p. | 10 i.p. |
| | active | active |

### Hot plate test

The hot plate test was used as an animal model of thermally-induced pain. The inhibitory activity measured on the time licking and biting induced by temperature (30 sec max at 55°C) indicates analgesic effect. Dose-dependent antinociceptive activity for the compound of example 2 at 10 and 30 mg/kg i.p. was observed. The compound of example 1 also demonstrated antinociceptive activity in the same test (Malmberg, A.B., and Bannon, A.W. Models of nociception: hot-plate, tail-flick, and formalin tests in rodents. Curr Protoc Neurosci, 2007, Ch. 8, Unit 8.9). Results for the compound of example 1 are presented in table 13.

The *in vivo* (formalin and hot plate test on mice) and *in vitro* (COX-1 and 5-LO inhibition) activity of compounds of formula (I) suggest that the antinociceptive activity of the compounds could be related to the inhibition of inflammatory pathways at the peripheral and/or central levels.

**Table 13. Analgesic activity of example 1 in the hot plate test**

| | | **Time Licking and biting (sec)** | |
|---|---|---|---|
| | **Dose (mg/kg i.p.)** | **Early phase (0-5 min)** | **Late phase (15-30 min)** |
| Vehicle | - | 70 | 250 |
| Example 1 | 1 | 57 | 200 |
| Example 1 | 5 | 45 | 150 |
| Example 1 | 10 | 38 | 150 |
| Example 1 | 30 | 30 | 20 |

### 5-Lipoxygenase Inhibition

The enzyme 5-Lipoxygenase (5-LO) catalyzes the first oxidation step in arachidonic acid metabolism, resulting in the synthesis of leukotrienes, which act primarily as mediators of inflammation and allergic reactions. 5-LO inhibitors are effective in the management of inflammatory diseases, chronic obstructive pulmonary disease (COPD), asthma, coronary artery disease, lung cancer, and Alzheimer's disease.

The 5-LO-inhibitory activity of compounds of the invention was determined using previously reported assays. Briefly, lipoxygenase activity was evaluated in human recombinant insect Sf9 cells using 3µM arachidonic acid as a substrate with a preincubation and incubation times of 15 and 5 min respectively at 25°C in a 50 mM Tris-HCl, pH 7.4, 2 mM CaCl₂, 1 mM ATP. The quantification method used was enzyme immunoassay determination of LTB4 and the significance criteria were established with a response equal or different of the maximum stimulation or inhibition (Noguchi, M. et al., Eur J Biochem, 1994, 222(2), 285-292; Percival, M.D. et al., Eur J Biochem, 1992, 210(1), 109-117).

Table 14 shows the inhibitory activity on 5-LO of selected compounds of the invention.

**Table 14. Inhibition of 5-LO at 10 µM**

| **Compound** | **% Inhibition** |
|---|---|
| Example 1 | 100 |
| Example 4 | 100 |
| Example 5 | 101 |
| Example 6 | 100 |
| Honokiol | 85 |
| Magnolol | 67 |

### Inhibition of Monoamine Transporters and Uptake

Monoamine transporters act in neurons by sequestering monoamines from nerve terminals. Norepinephrine transporter (NET) inhibitors produce an increase of norepinephrine (NE) in the brain and have been investigated for the treatment of several diseases including depression and anxiety.

Serotonin transporter (SERT) inhibitors increase extracellular concentrations of serotonin (5-HT) and amplify signals sent by serotonin neurons. Agents that block SERT have been studied for the treatment of several diseases including depression and anxiety.
Inhibitors of monoamine transporters have been tested for NET (Pacholczyk, T. et al. Nature, 1991, 350, 350-354) and SERT (Tatsumi, M. et al. Eur J Pharmacol, 1999, 368, 277-283). Norepinephrine transport was assessed in human recombinant CHO cells using [³H]nisoxetine as a ligand at 1 nM after 60 min of incubation at 22°C, and a scintillation method as a method of detection. Serotonine transport was assessed in human recombinant CHO cells using [³H]imipramine as a ligand at 2 nM after 60 min of incubation at 22°C, and a scintillation counting as a method of detection.

Antidepressants are complex drugs with a wide variety of pharmacological actions including NE and 5-HT reuptake inhibition. Chronic pain and depression are frequent comorbid disorders. Each can predispose to the other, and their coexistence often complicates the treatment of either one or both disorders. Antidepressants may be useful for the treatment of chronic pain syndromes.

Inhibition of serotonin uptake has been tested in rat brain synaptosomes using [³H]serotonin (0.2 µCi/ml) after 15 min of incubation at 37°C and measuring the incorporation of [³H]serotonin into synaptosomes by scintillation counting (Perovic, S. and Muller, W.E.G. Arzneim-Forsch. Drug Res., 1995, 45, 1145-1148).

Compounds of formula (I) are active when tested for inhibition of monoamine transporters and uptake.

Table 15 shows the inhibitory activity of the compound of example 3 against NET, SERT, NE and 5-HT uptake.

**Table 15. Inhibition of monoamine transporters and uptake**

| | **% Inhibition (5.0 µM)** | | | |
|---|---|---|---|---|
| **Compound** | **NET** | **NE Uptake** | **SERT** | **5-HT Uptake** |
| Example 3 | 87 | 85 | 73 | 52 |

### 5-HT_{2A} Receptor Antagonism

The 5-HT_{2A} receptor belongs to the G-protein-coupled receptor (GPCR) target class linked to anxiety and depression. Using human recombinant HEK-293 cells, the binding of compounds of formula (I) to this receptor was determined. The ligand used was [³H]ketanserin at 0.5 nM and the quantification of the activity was assessed by scintillation counting after 60 min at 22°C (Bonhaus, et al. Br J Pharmacol, 1995, 115, 622-628).

**Table 16. Inhibition of 5-HT_{2A} binding**

| **Compound** | **% Inhibition (5.0 µM)** |
|---|---|
| Example 3 | 86 |

### Gonadotrophin-Releasing Hormone Receptor Agonism

Gonadotrophin-releasing hormone receptors (GnRH, LH-RH)R are a family of G protein-coupled receptors for GnRH, which promotes the secretion of luteinizing hormone (LH) and follicle-stimulating hormone (FSH). The activation of this receptor promotes, through estrogen receptors (ER) located in the cytosol, an increase in gene transcription and activation of tumor suppressor genes, DNA repair, cell division and cell proliferation. GnRH agonists are useful in breast, prostate and ovarian cancer, endometriosis, melanoma, rheumatoid arthritis and Alzheimer's disease.
Table 17 presents the results from radioligand binding studies for GnRH for selected compounds using human recombinant Chem-1 cells (Halmos G, Schally AV, Pinski J, Vadillo-Buenfill M, Groot K. Down regulation of pituitary receptors for luteinizing hormone-releasing hormone (LH-RH) in rats by LH-RH antagonist Cetrorelix. Prco Natl Acad Sci USA 1996;93(6):2398-2402). The specific and non specific ligands used were [¹²⁵l] [D-Trp⁶]-LH-RH (0.05 nM) and [D-Trp⁶]-LH-RH (1 mM) and after 60 min of incubation at 25°C in a 50 mM HEPES, pH 7.5, 5 mM MgCl₂, 1 mM CaCl₂, and 0.2% BSA the activity was measured by radioligand binding.

**Table 17. GnRH radioligand binding assay**

| **Compound** | **% Inhibition at 10 µM** |
|---|---|
| Example 1 | 50 |
| Example 2 | 33 |
| Example 5 | 37 |

### Pharmacokinetics

Absorption, distribution and metabolism studies in vitro with selected examples were performed in order to determine pharmacokinetic parameters such as permeability (or absorption constant), intrinsic clearance (or metabolic clearance), and bioavailability. Physicochemical properties such as water solubility, pKa, log P, and log D_{octanol/water}, and in vitro experimental studies such as permeability in Caco2 cells, and protein binding in the mouse, stability in murine microsomes, chemical stability at different pH and stability in plasma were evaluated for the pharmacokinetic prediction in mice.

### Pharmaceutical Compositions

The present invention also includes pharmaceutically active compositions comprising compounds of formula (I) and a physiological carrier. The active compositions can be administered either orally, subcutaneously, parenterally, locally (ointments, creams, powders), as drops or as a nasal or buccal spray or directly injected into tumors.

The following examples of pharmaceutical compositions are offered with the understanding that they are in no way limiting of the invention.

### Formulation Example 1: Preparation of conjugates with magnetite nanoparticles.

Compounds of formula (I) are good candidates for melanoma chemotherapy. Nanoparticles may provide a good platform for coadministering anticancer therapies. Magnetite nanoparticles have been shown to possess great potential for developing thermo-therapies. Magnetite particles (Fe₃O₄, average particle size 10 nm) are coated with aminosilane and conjugated with the compound of example 1 via meleimide cross-linkers. The resulting product can be suspended in water and conveniently applied.

### Formulation Example 2: Compositions for parenteral administration.

These compositions may comprise physiologically acceptable sterile aqueous or non-aqueous solutions, emulsions, suspensions and powders for reconstitution as injectable solutions.

The compound of example 1 in powder form is diluted in phosphate buffered saline consisting of polysorbate 80, potassium chloride, monobasic phosphate, sodium chloride, dibasic sodium phosphate and water for injection prepared to the desired volume.

Liposome injections for intrathecal use are prepared in a sterile, injectable suspension of compounds of formula (I), encapsulated in multi-vesicular lipid-based particles.
The compound of example 1 is mixed with PEG400 (polyethylene 400)/5 % aqueous dextrose in a volume of 7:3 and diluted with water for injection 20 mg/mL.

The compounds of example 1, example 2 and example 5, 2 mg are dissolved in 20 µL of DMSO and then diluted with 2 mL of water for injection.

### Formulation Example 3: Tablets and capsules for oral administration

Tablets are prepared containing as inactive ingredients lactose, magnesium stearate, hydroxypropylmethylcellulose, polyethylene glycol, povidone, sodium starch glycolate, and titanium dioxide.

Capsules are prepared containing as inactive ingredients talcum, sodium lauryl sulfate, celluloid silicon dioxide, magnesium stearate, titanium dioxide (E171), hard gelatin capsule, black ink, propyleneglycol and shellac.

### Formulation Example 4: Creams for topical administration

Examples are incorporated in an off-white oil in water, vanishing cream base consisting of isostearic acid, cetyl alcohol, stearyl alcohol, white petrolatum, polysorbate 60, sorbitan monostearate, glycerin, xanthan gum, purified water, benzyl alcohol, methylparaben and propylparaben.

### Formulation Example 5: Transdermal Formulations

Pluronic F127 is a bifunctional block copolymer surfactant terminating in primary hydroxyl groups. It is relatively nontoxic and has been used for transdermal drug delivery. Transdermal drug delivery system formulations of example 5 based on Pluronic F127 are prepared and show good therapeutic response.

### Combination Therapy

The use of compounds of the invention together or in combination with other antitumor agents has been shown to be effective in treating many cancers. Combination therapy helps overcome chemotherapy resistance in many forms of cancer. Combination therapy includes the use of two or more compounds of the invention, e.g. compounds of examples 1 and 8, or the use of one compound of the invention in combination with other known antineoplastic agents.

## Claims

1. A compound of formula (I): 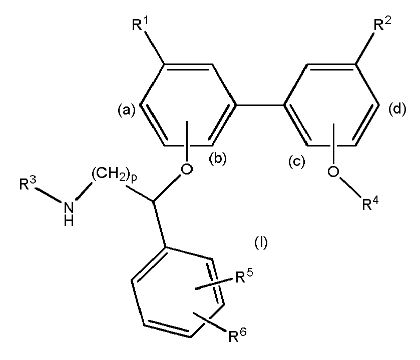 wherein R¹ and R² are independently selected from the group consisting of C₁-C₈ alkyl, C₂-C₈ alkenyl, and C₂-C₈ alkynyl;
R³ is a C₁-C₄ alkyl group;
p is an integer selected from 1, 2, 3 and 4;
both R⁵ and R⁶ are independently selected from hydrogen atom, C₁-C₈ alkyl, - OH, halogen atoms and -CF₃; or R⁵ and R⁶ together with the phenyl ring to which they are attached form a benzodioxole group;
R⁴ is a group selected from:
a) hydrogen atom, C₁-C₈ alkyl, C₂-C₈ alkenyl, and C₂-C₈ alkynyl, all optionally substituted by one or more groups selected from halogen, -N(R^{a})(R^{b}), - O(C=O)-C₁-C₈ alkylene-N(R^{a})(R^{b}), -O(C=O)-C₁-C₈ alkenylene-N(R^{a})(R^{b}), C₃-C₂₀ cycloalkyl, C₄-C₁₂ cycloalkenyl, -NH-C(=NH)-NH₂, -C(=NH)-NH₂, - (CH₂)ₙ-SO₂-R, -N(R^{a})-S(=O)₂-R, -S(=O)₂-N(R^{a})(R^{b}), -N(R^{a})-C(=O)O-R, - S(=O)-R, -S(=O)₂-R, -N(C₁-C₈ alkyl)-C(=O)N(R^{a})(R^{b}), -NHOH, C₆-C₁₀ aryl, heterocyclyl, and heteroaryl;
b) C₃-C₂₀ cycloalkyl, C₄-C₁₂ cycloalkenyl and C₆-C₁₀ aryl, all optionally substituted by one or more groups selected from halogen, -OH, -CF₃, -C₁-C₈ alkylene-N(R^{a})(R^{b}), -O(C=O)-C₁-C₈ alkylene-N(R^{a})(R^{b}), C₃-C₂₀ cycloalkyl, C₄-C₁₂ cycloalkenyl, -NH-C(=NH)-NH₂, -C(=NH)-NH₂, -(CH₂)ₙ-S(=O)₂-R, - N(R^{a})S(=O)₂-R, -S(=O)₂-N(R^{a})(R^{b}), -N(R^{a})-C(=O)O-R, -S(=O)-R, -S(=O)₂-R, -N(C₁-C₈ alkyl)-C(=O)N(R^{a})(R^{b}), -NHOH, C₆-C₁₀ aryl, heterocyclyl and heteroaryl;
c) heterocyclyl and heteroaryl, both optionally substituted by one or more groups selected from -OH, -CF₃, C₁-C₈ alkyl, C₂-C₈ alkenyl, C₂-C₈ alkynyl, C₃-C₂₀ cycloalkyl, C₄-C₁₂ cycloalkenyl, halogen, -C₁-C₈ alkylene-N(R^{a})(R^{b}), -(CH₂)ₙ-S(=O)₂-R, C₆-C₁₀ aryl-C₁-C₆ alkyl and phenyl optionally substituted by one or more groups selected from C₁-C₈ alkyl, perfluoro-C₁-C₈ alkyl, C₁-C₈ alkoxy, hydroxy-C₁-C₈ alkyl, halogen, -OH, -S-R, or benzyloxy;
d) -C(=O)N(R^{a})(R^{b});
e) -C(=O)OR^{c};
f) -C(=O)R^{c};
g) -P(=O)(OR^{d})(OR^{e});
h) -P(=O)(OR^{d})-CH₂₋P(=O)(OR^{d})(OR^{e});
i) a sugar moiety selected from: 1-O-β-D-glucopyranosyl, 1-O-β-D-galactopyranosyl, and 1-O-α-D-glucopyranosyl-a-D-glucopyranosyl;
j) an aminoacid moiety derived from a naturally occurring aminoacid;
n is an integer selected from 1 to 6;
wherein R is a hydrogen atom or a group selected from C₁-C₈ alkyl, C₂-C₈ alkenyl, C₂-C₈ alkynyl, C₃-C₂₀ cycloalkyl, C₆-C₁₀ aryl, and C₆-C₁₀ aryl-C₁-C₈ alkyl;
R^{a} and R^{b} are independently selected from hydrogen atom, C₁-C₈ alkyl, C₂-C₈ alkenyl, C₂-C₈ alkynyl, C₃-C₂₀ cycloalkyl, C₄-C₁₂ cycloalkenyl, C₆-C₁₀ aryl, heterocyclyl, heteroaryl, and C₂-C₈ acyl all of them optionally substituted by one or more groups selected from halogen, C₁-C₈ alkoxy, -S-C₁-C₈ alkyl, C₆-C₁₀ aryl, heteroaryl, -NH₂, -NH-C₁-C₈ alkyl or -N(C₁-C₈ alkyl)₂, -S(=O)₂-C₁-C₈ alkyl, - S(=O)-C₁-C₈ alkyl, -C(=O)OH, -C(=O)O-C₁-C₈ alkyl and -C(=O)-C₁-C₈ alkyl;
or R^{a} and R^{b} together with the N to which they are attached form a saturated, monounsaturated or polyunsaturated 5,6-membered nitrogenated heterocycle, which may optionally contain another heteroatom selected from O, N, and S; R^{c}, R^{d}, and R^{e} are independently selected from hydrogen atom, C₁-C₈ alkyl, C₂-C₈ alkenyl, C₂-C₈ alkynyl, C₂-C₈ acyl, C₃-C₂₀ cycloalkyl, C₄-C₁₂ cycloalkenyl, C₆-C₁₀ aryl, heterocyclyl and heteroaryl all of them optionally substituted by one or more groups selected from halogen, -OH, C₁-C₈ alkoxy, -S-C₁-C₈ alkyl, C₆-C₁₀ aryl, heteroaryl, -NH₂, -NH-C₁-C₈ alkyl or -N(C₁-C₈ alkyl)₂, -S(=O)-C₁-C₈ alkyl, - S(=O)-C₁-C₈ alkyl, -C(=O)OH, -C(=O)O-C₁-C₈ alkyl and -C(=O)-C₁-C₈ alkyl;
wherein either the substituent -O-CH(substituted phenyl)-(CH₂)p-NH-R³ occupies the carbon atom marked as (a) while the substituent -OR⁴ occupies the carbon atom marked as (c), or the substituent -O-CH(substituted phenyl)-(CH₂)ₚ-NH-R³ occupies the carbon atom marked as (b) while the substituent - OR⁴ occupies either the carbon atom marked as (c) or the carbon atom marked as (d);
or a pharmaceutically acceptable salt or stereoisomer thereof.

2. A compound of formula (I) according to claim 1 wherein the compound is in salt form, the secondary amino group of the group -O-CH(phenyl)-(CH₂)ₚ-NH-R³ is further protonated (-NH₂⁺-) and the compound further comprises a pharmaceutically acceptable counter-anion.

3. A compound according to anyone of claims 1 to 3 wherein R⁴ is selected from:
a) hydrogen atom, C₁-C₈ alkyl, C₂-C₈ alkenyl and C₂-C₈ alkynyl, all of which are optionally substituted by one or more groups selected from halogen, - N(R^{a})(R^{b}), -O(C=O)-C₁-C₈ alkylene-N(R^{a})(R^{b}), C₃-C₂₀ cycloalkyl, -NHOH, C₆-C₁₀ aryl, heterocyclyl and heteroaryl;
b) C₃-C₂₀ cycloalkyl, C₄-C₁₂ cycloalkenyl and C₆-C₁₀ aryl, all optionally substituted by one or more groups selected from halogen, -C₁-C₈-alkylene-N(R^{a})(R^{b}), -O(C=O)-C₁-C₈ alkylene-N(R^{a})(R^{b}), C₃-C₂₀ cycloalkyl, -NHOH, C₆-C₁₀ aryl, heterocyclyl and heteroaryl;
c) heterocyclyl and heteroaryl, both optionally substituted by one or more groups selected from C₁-C₈ alkyl, C₃-C₂₀ cycloalkyl, halogen, -C₁-C₈ alkylene-N(R^{a})(R^{b}) and phenyl optionally substituted by one or more groups selected from C₁-C₈ alkyl, -CF₃, C₁-C₈ alkoxy, hydroxy-C₁-C₈ alkyl, halogen and -OH;
d) -C(=O)N(R^{a})₍R^{b});
e) -C(=O)OR^{c};
f) -C(=O)R^{c};
g) -P(=O)(OR^{d})-CH₂-P(=O)(OR^{d})(OR^{e});
h) a sugar moiety selected from: 1-O-β-D-glucopyranosyl, 1-O-β-D-galactopyranosyl, and 1-O-α-D-glucopyranosyl-a-D-glucopyranosyl;
R^{a} and R^{b} are independently selected from hydrogen atom, C₁-C₈ alkyl, C₃-C₂₀ cycloalkyl, C₆-C₁₀ aryl, and C₂-C₈ acyl all of them optionally substituted by one or more groups selected from halogen, C₁-C₈ alkoxy, -S-C₁-C₈ alkyl, C₆-C₁₀ aryl and heteroaryl;
or R^{a} and R^{b} together with the N to which they are attached form a saturated, monounsaturated or polyunsaturated 5,6-membered nitrogenated heterocycle, which may optionally contain another heteroatom selected from O, N, and S; wherein R^{c}, R^{d}, and R^{e} are independently selected from hydrogen atom, C₁-C₈ alkyl, C₂-C₈ alkenyl, C₃-C₂₀ cycloalkyl, C₆-C₁₀ aryl, heterocyclyl, and heteroaryl all of them optionally substituted by one or more groups selected from halogen, -OH, C₁-C₈ alkoxy, -S-C₁-C₈ alkyl, -C(=O)O-C₁-C₈ alkyl, C₆-C₁₀ aryl and heteroaryl;

4. A compound according to anyone of claims 1 to 3 wherein p has a value of 2 and R³ is a methyl group.

5. A compound according to anyone of claims 1 to 4 wherein R¹ and R² are independently selected from the group consisting of allyl, 2-propenyl, n-propyl, methyl, isopropyl, tert-butyl, and ethynyl.

6. A compound according to claim 5 wherein R¹ and R² are independently selected from the group consisting of allyl and n-propyl.

7. A compound according to claim 6 wherein R¹ and R² are allyl.

8. A compound according to anyone of claims 2 to 7 wherein R⁴ is selected from:
a) hydrogen atom, C₁-C₈ alkyl and C₂-C₈ alkenyl, both of them optionally substituted by one or more groups selected from halogen, -N(R^{a})(R^{b}), - O(C=O)-C₁-C₈ alkylene-N(R^{a})(R^{b}), C₃-C₂₀ cycloalkyl, -NHOH, C₆-C₁₀ aryl, heterocyclyl and heteroaryl;
b) C₃-C₂₀ cycloalkyl and C₆-C₁₀ aryl, both optionally substituted by one or more groups selected from halogen, -C₁-C₈-alkylene-N(R^{a})(R^{b}), -O(C=O)-C₁-C₈ alkylene-N(R^{a})(R^{b}), C₃-C₂₀ cycloalkyl, -NHOH, C₆-C₁₀ aryl, heterocyclyl and heteroaryl;
c) heterocyclyl and heteroaryl, both optionally substituted by C₁-C₈ alkyl, C₃-C₂₀ cycloalkyl, halogen and phenyl optionally substituted by one or more groups selected from C₁-C₈ alkyl, halogen and -OH;
d) -C(=O)N(R^{a})(R^{b});
e) -C(=O)R^{c};
f) -P(=O)(OR^{d})-CH₂-P(=O)(OR^{d})(OR^{e});
g) a sugar moiety selected from: 1-O-β-D-glucopyranosyl, and 1-0-α-D-glucopyranosyl-α-D-glucopyranosyl;
wherein R^{a}, R^{b}, R^{c}, R^{d} and R^{e} are as defined in claim 3

9. A compound according to claim 8, wherein R⁴ is selected from:
a) hydrogen atom, C₁-C₈ alkyl and C₂-C₈ alkenyl, both optionally substituted by one or more groups selected from halogen, -N(R^{a})(R^{b}), -O(C=O)-C₁-C₈ alkylene-N(R^{a})(R^{b}), C₃-C₂₀ cycloalkyl, -NHOH and C₆-C₁₀ aryl;
b) C₃-C₂₀ cycloalkyl and C₆-C₁₀ aryl, both optionally substituted by one or more groups selected from halogen, -C₁-C₈-alkylene-N(R^{a})(R^{b}), -O(C=O)-C₁-C₈ alkylene-N(R^{a})(R^{b}), C₃-C₂₀ cycloalkyl, -NHOH and C₆-C₁₀ aryl;
c) -C(=O)N(_{R}^{a})(_{R}^{b}_{);}
d) -C(=O)R^{c};
e) -P(=O)(OR^{d})-CH₂-P(=O)(OR^{d})(OR^{e});
f) a sugar moiety selected from: 1-O-α-D-glucopyranosyl, and 1-O-α-D-glucopyranosyl-α-D-glucopyranosyl;
wherein R^{a}, R^{b}, R^{c}, R^{d} and R^{e} are as defined in claim 3

10. A compound according to claim 9, wherein R⁴ is selected from:
a) hydrogen atom
b) C₁-C₄ alkyl optionally substituted by one or more halogen atoms;
c) -C(=O)R^{c};
wherein R^{c} is a C₁-C₈ alkyl.

11. A compound according to claim 10, wherein R⁴ is selected from hydrogen atom, methyl and ethyl.

12. A compound according to claim 8 wherein R⁴ is selected from:
a) hydrogen atom, C₁-C₈ alkyl and C₂-C₈ alkenyl, both optionally substituted by one or more groups selected from -N(R^{a})(R^{b}), C₆-C₁₀ aryl, heterocyclyl and heteroaryl;
b) C₃-C₂₀ cycloalkyl and C₆-C₁₀ aryl, both optionally substituted by one or more groups selected from C₆-C₁₀ aryl, heterocyclyl and heteroaryl;
c) heterocyclyl and heteroaryl, both optionally substituted by C₁-C₈ alkyl, C₃-C₂₀ cycloalkyl, halogen and phenyl optionally substituted by one or more groups selected from C₁-C₈ alkyl, halogen and -OH;
wherein R^{a} and R^{b} are as defined in claim 2.

13. A compound according to claim 1, which is selected from:
• 5,5'-Diallyl-2'-trifluoromethoxy-2-(3-methylamino-1-phenylpropoxy)biphenyl.
• 5,5'-Diallyl-2-(4-methoxy-4-oxobut-2-enoyloxy)-2'-(3-methylamino-1-phenylpropoxy)biphenyl.
• 5,5'-Dimethyl-2-methoxy-2'-(3-methylamino-1-phenylpropoxy)biphenyl.
• 2'-[3-(Methylamino)-1-phenylpropoxy]-5,5'-di(prop-1-enyl)biphenyl-2-ol.
• 2'-(3-Methylamino-1-phenylpropoxy)-3,5'-di(prop-1-enyl)biphenyl-4-ol.
• 2-Methoxy-4'-(3-methylamino-1-phenylpropoxy)-3',5-di(prop-1-enyl)biphenyl.
• 5,5'-Diallyl-2'-[3-(methylamino)-1-phenylpropoxy]biphenyl-2-ol.
• 5,5'-Diallyl-2-methoxy-2'-[3-(methylamino)-1-phenylpropoxy]biphenyl.
• 5,5'-Diallyl-2-ethoxy-2'-[3-(methylamino)-1-phenylpropoxy]biphenyl.
• 3,5'-Diallyl-2'-[3-(methylamino)-1-phenylpropoxy]biphenyl-4-ol.
• 3',5-Diallyl-4'-[3-(methylamino)-1-phenylpropoxy]biphenyl-2-ol.
• 4'-[3-(Methylamino)-1-phenylpropoxy]-3',5-dipropylbiphenyl-2-ol.
• 3',5-Diallyl-4'-ethoxy-2-[3-(methylamino)-1-phenylpropoxy]biphenyl mixture with 3 , 5 '-diallyl-2'-ethoxy-4-[3-(methylamino)-1-phenylpropoxy]biphenyl.
• 5,5'-Diallyl-2'-[3-(methylamino)-1-phenylpropoxy]biphenyl-2-yl 2-propylpentanoate.

14. A pharmaceutical composition comprising a compound as defined in anyone of claims 1 to 13 and one or more pharmaceutically acceptable carriers.

15. A compound as defined in anyone of claims 1 to 13 for use as a medicament.

16. A compound as defined in any one of claims 1 to 13 for use in suppressing tumor growth and/or preventing recurrence or metastasis or in the treatment of a cancer selected from the group consisting of brain, bone, colon, ovary, pancreas, prostate, skin, lung, and head and neck cancer, melanoma and hematological malignancies or in the treatment of nononcological indications such as skin inflammation, atopic dermatitis, or actinic keratosis or in the treatment of autoimmune disorders such as multiple sclerosis, psoriatic arthritis, rheumatoid arthritis, scleroderma, systemic lupus, erythematosus, dermatomyositis, Sjögren's syndrome and Behçet's disease or in the treatment of pain, selected from the group consisting of chronic, cancer, postoperative and neuropathic pain or for the simultaneous treatment of cancer and cancer-related pain and inflammation.

17. A combination comprising a compound as defined in anyone of claims 1 to 13 and another anticancer agent.

18. A combination as defined in claim 17 wherein the additional anticancer agent is selected from the group consisting of paclitaxel, cisplatin, carboplatin, trastuzumab, lapatinib, tamoxifen, letrozole, gefitinib, anastrozole, everolimus, irinotecan, or temozolomide.

19. A combination comprising a compound as defined in anyone of claims 1 to 13 and another active ingredient selected from the group consisting of *O*⁶-benzylguanine, glucosamine, valproic acid, butyric acid, metformin, or dichloroacetate.
